# EUROPEAN PATENT SPECIFICATION

(11) **EP 0 500 785 B1**
(45) Date of publication and mention of the grant of the patent: **17.04.2002**
(21) Application number: 91900596.7
(22) Date of filing: 02.11.1990
(51) Int. Cl.: C07K 14/47, A61K 38/17, A61K 39/35

(54) **A HUMAN T CELL REACTIVE FELINE PROTEIN (TRFP) ISOLATED FROM HOUSE DUST AND USES THEREFOR**
AUS HAUSSTAUB ISOLIERTES T-ZELLREAKTIVES KATZENPROTEIN, UND DESSEN VERWENDUNGEN
PROTEINE DE FELIN REAGISSANT AVEC DES LYMPHOCYTES T HUMAINS (PFRL), ISOLEE A PARTIR DE POUSSIERES MENAGERES ET SON USAGE

(30) Priority: 03.11.1989 US 431565
(43) Date of publication of application: 02.09.1992
(73) Proprietor: IMMULOGIC PHARMACEUTICAL CORPORATION, Cambridge Massachusetts 02139 (US)
(72) Inventor: GEFTER, Malcolm, L., Weston, MA 02193 (US); GARMAN, Richard, D., Somerville, MA 02114 (US); GREENSTEIN, Julia, L., Newton, MA 02159 (US); KUO, Mei-chang, Winchester, MA 01890 (US); ROGERS, Bruce, L., Cambridge, MA 02139 (US); BRAUER, Andrew, W., Salem, MA 01970 (US); GRIFFITH, Irwin. J., North Reading, MA 01864 (US); MORGENSTERN Jay, Boston, MA 02116 (US); BOND, Julian, Weymouth MA 02188 (US)
(74) Representative: Price, Vincent Andrew
(86) International application number: US9006548
(87) International publication number: WO9106571

(56) References cited:
- US-A- 4 163 778
- Dialog Information Services, File 155: Medline, NLM accession number 88116696, Chapman M.D. et al: "Monoclonal antibodies to the major *feline* *allergen* Fel d I. II. Single step affinity purification of Fel d I, N-terminal sequence analysis, and development of a sensitive two-site immunoassay to assess Fel d I exposure", & J Immunol (UNITED STATES) Feb 1 1988, 140 (3) p812-818.
- Dialog Information Services, File 155: Medline, NLM accession number 84110579, Didierlaurent A. et al: "Comparative study on *cat* *allergens* from fur and saliva", & Int Arch Allergy Appl Immunol 1984, 73 (1) p27-31.

## Description

Genetically predisposed individuals, who make up about 10% of the population, become hypersensitized (allergic) to antigens from a variety of environmental sources to which they are exposed. Those antigens that can induce immediate and/or delayed types of hypersensitivity in people are called allergens. King, T.P., Adv. Immun., 23:77-105 (1976). The symptoms of hay fever, asthma and hives are forms of allergy which can be caused by a variety of allergens, such as products of grasses, trees, weeds, animal dander, insects, food, drugs and chemicals. The antibodies involved in allergy belong primarily to the IgE class of immunoglobulins. IgE binds to mast cells and basophils. Upon combination of a specific allergen with IgE bound to mast cells, the IgE is cross-linked on the cell surface, resulting in the physiological effects of IgE-antigen interaction. Degranulation results in release of, among other substances, histamine, heparin, a chemotactic factor for eosinophilic leukocytes and the leukotrienes, C4, D4 and E4, which cause prolonged constriction of bronchial smooth muscle cells. Hood, L.E. et al., Immunology, (2nd ed.), pp460-462, The Benjamin/Cumming Publishing Co., Inc. (1984). These released substances are the mediators which result in allergic symptoms caused by combination of IgE with a specific allergen. Through them, the effects of an allergen are manifested. Such effects may be systemic or local in nature, depending on the route by which the antigen entered the body and the pattern of deposition of IgE and mast cells. Local manifestations generally occur on epithelial surfaces at the location at which the allergen entered the body. Systemic effects can include anaphylaxis (anaphylactic shock), which is the result of an IgE-basophil response to circulating (intravascular) antigen.

It has been estimated that there are approximately 10 million cat allergic individuals in the United States. Ohman, J.L., and Sundin, B., Clin. Rev. Allergy, 5:37-47 (1987). An allergen of particular concern for many people is the feline skin and salivary gland allergen of the domestic cat Felis domesticus allergen I (Fel d I), also referred to as allergen I, cat 1 and antigen 4. Fel d I has been described as an acidic non-covalently linked homodimer of approximately 39,000 molecular weight on size exclusion HPLC, and 17,000 under nonreducing conditions on gel electrophoresis. Chapman, M.D., et al. J. Immunology, 140(3):812-818 (1988). Chapman and co-workers also describe a single step procedure for the purification of Fel d I from crude house dust extract with a high Fel d I content (50 U/ml) using monoclonal antibody affinity chromatography. In addition, they determined the amino acid composition and partial amino acid sequence of Fel d I. Fel d I has also been described as a 35,000 molecular weight dimer of two noncovalently linked 18,000 molecular weight subunits, which occurs in three isoallergenic forms (pI 3.5 to 4.1). Ohman, J.L., et al., J. Allergy Clin. Immunol., 52:231 (1973); Ohman, J.L., et al., J. Immunol., 113:1668 (1974); Leiterman, K., and Ohman, J.L., J. Allergy Clin. Immunol., 74:147 (1984).

Exposure to cat allergen can occur as a result of exposure to the animal or contact with house dust which contains cat allergens. These allergens have been examined in saliva, skin scrapings, cat wash, serum, salivary glands, cat hair, cat dandruff and house dust.

Despite the considerable attention allergic responses to cat allergens and cat allergens themselves have received, definition or characterisation of the structure and components of the Fel d I allergen believed to be responsible for the adverse effects on cat-sensitive individuals is far from complete and current desensitisation therapy involves treatment with a complex, ill-defined animal dander extract.

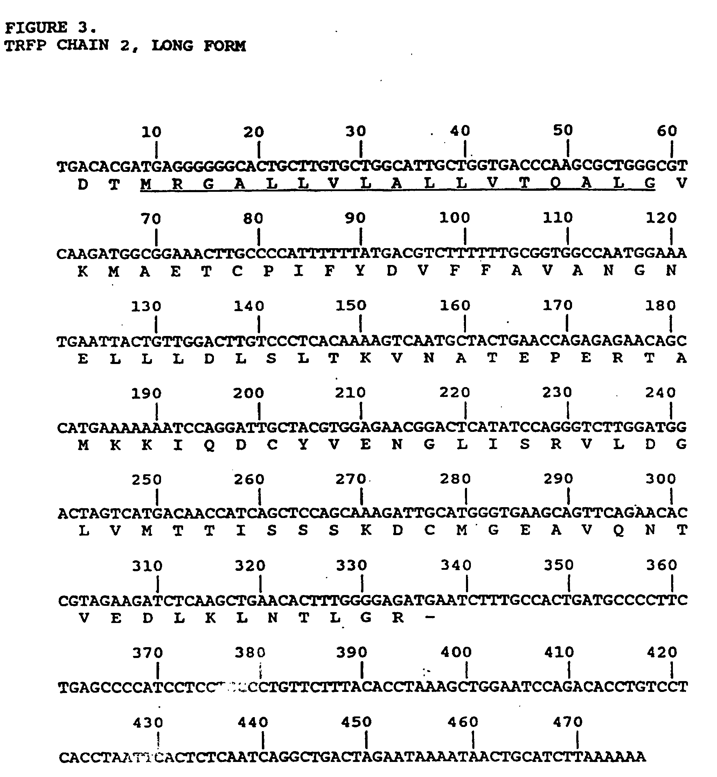

Accordingly, in a first aspect the present invention provides a composition for use in medicine, comprising a first polypeptide that:
a) has an amino acid sequence as shown in any of Figures 3, 4, 5 or 7, or
b) has at least part of a sequence as described in a) above and has an epitope in common therewith, or
c) is a modified form of a polypeptide as described in a) or b) above, but has at least one epitope in common therewith;
wherein said composition can be used to reduce an allergic response to a cat antigen in an individual sensitive to said antigen.

Preferably an embodiment of a composition for use in medicine according to the invention comprises first polypeptide as shown in Table 3.

Preferably an embodiment of a composition for use in medicine according to the invention comprises a mixture of said first polypeptides.

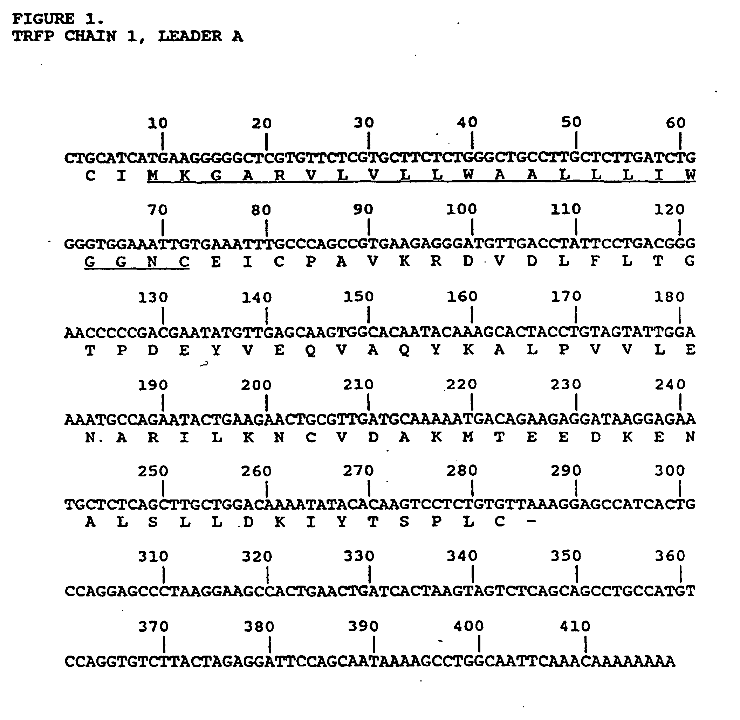

Preferably an embodiment of a composition for use in medicine according to the invention comprises a second polypeptide that is different from the first polypeptide and that:
has an amino acid sequence as shown in Figure 1, Figure 2 or Figure 6, or
has at least part of a sequence as described in a) above and has an epitope in common therewith, or
is a modified form of a polypeptide as described in a) or b) above, but has at least one epitope in common therewith.

Preferably an embodiment of a composition for use in medicine according to the invention comprises a second polypeptide as described in a) or b) above.

Preferably an embodiment of a composition for use in medicine according to the invention comprises a second polypeptide as shown in Table 2.

Preferably an embodiment of a composition for use in medicine according to the invention comprises a mixture of said second polypeptides, or a mixture of said first and second polypeptides.

Preferably an embodiment of a composition for use in medicine according to the invention comprises first and second polypeptides covalently linked in the form of a heterodimer.

Preferably an embodiment of a composition for use in medicine according to the invention has a stimulation index of at least approximately 4.0 or a positivity index of at least approximately 250, wherein the stimulation index is defined as the ratio of measured proliferation of T cells and antigen presenting cells from a responding individual when exposed to antigen to the measured proliferation of said cells in the absence of antigen, and wherein the positivity index is defined as the average stimulation index of a population of responding individuals tested multiplied by the percentage of individuals in the population exhibiting a positive response.

In a second aspect the present invention provides a polypeptide that:
a) has an amino acid sequence as shown in any of Figures 3, 4, 5 or 7, or
b) has at least part of a sequence as described in a) above and has an epitope in common therewith, or
c) is a modified form of a polypeptide as described in a) or b) above, but has at least one epitope in common therewith.

Preferably, an embodiment of a polypeptide according to the invention is as shown in Table 3.

Preferably, an embodiment of a polypeptide according to the invention has a stimulation index of at least approximately 4.0 or a positivity index of at least approximately 250 wherein the stimulation index is defined as the ratio of measured proliferation of T cells and antigen presenting cells from a responding individual when exposed to antigen to the measured proliferation of said cells in the absence of antigen, and wherein the positivity index is defined as the average stimulation index of a population of responding individuals tested multiplied by the percentage of individuals in the population exhibiting a positive response.

Preferably, an embodiment of a polypeptide according to the invention is for use in medicine.

In a third aspect the present invention provides use of a polypeptide or of a composition according to the invention in the preparation of a medicament for treating a cat allergy.

In a fourth aspect the present invention provides a polypeptide or a composition according to the invention for use in diagnosis.

In a fifth aspect the present invention provides an *ex vivo* method of detecting sensitivity in an individual to a cat allergen, comprising combining a blood sample obtained from the individual with a polypeptide or a composition according to the invention.

In a sixth aspect the present invention provides a method comprising preparing a polypeptide according to the invention by recombinant means.

Preferably, an embodiment of a method according to the invention further comprises preparing a second polypeptide according to the invention by recombinant means.

Preferably, an embodiment of a method according to the invention comprises expressing said polypeptide or polypeptides in *E. coli*.

Preferably, an embodiment of a method according to the invention is used to produce a heterodimer as described above.

In a seventh aspect the present invention provides DNA encoding a polypeptide according to the invention.

Preferably an embodiment of DNA according to the invention is cDNA.

In an eighth aspect the present invention provides DNA capable of hybridizing to DNA according to a seventh aspect of the invention.

Figure 1 is the DNA sequence and deduced amino acid sequence of TRFP, chain 1, leader A (underlined).

Figure 2 is the DNA sequence and deduced amino acid sequence of TRFP, chain 2, leader B (underlined).

Figure 3 is the DNA sequence and deduced amino acid sequence of TRFP, chain 2, long form (476 nucleotides).

Figure 4 is the DNA sequence and deduced amino acid sequence of TRFP, chain 2, short form (469 nucleotides).

Figure 5 is the DNA sequence and deduced amino acid sequence of TRFP, chain 2, truncated form (465 nucleotides).

Figure 6 is the protein sequence of TRFP, chain 1, with leader A and leader B (top two lines). The deduced amino acid sequence of the leaders and for chain 1 (C1) were obtained by sequencing cDNA and the protein sequence for chain 1 (PRO) was determined by protein sequencing methods, with amino acid numbering based upon the first amino acid determined by protein sequencing methods. The presumed initiator methionine in each leader sequence is in bold type. (-) symbolises complete agreement or identity with the amino acid residue listed above the (-).

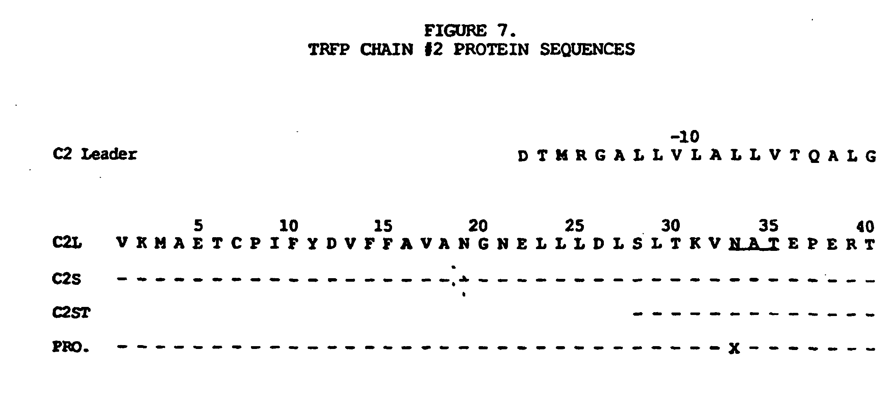

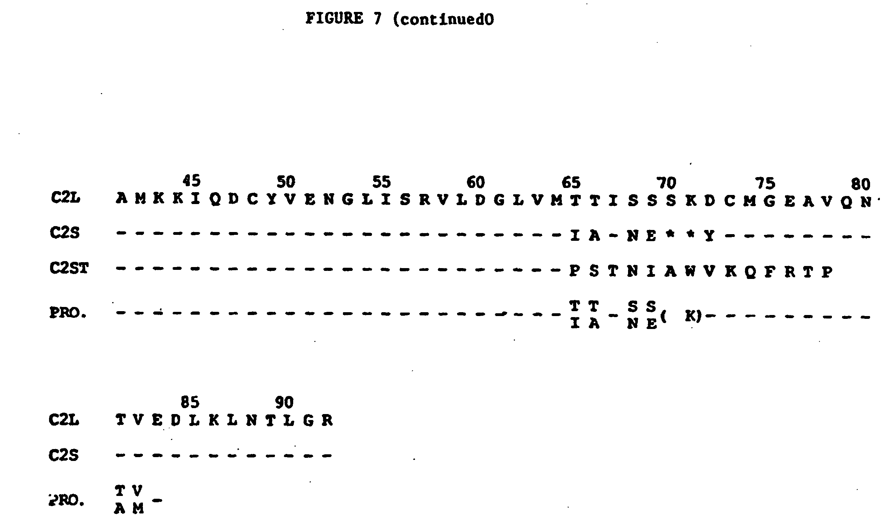

Figure 7 is the protein sequence of TRFP, Chain 2 with the leader sequence. The deduced amino acid sequence of the leader and for Chain 2 (C2) were obtained by sequencing cDNA. The protein sequence for Chain 2 (PRO) was determined by protein sequencing methods, with amino acid numbering based upon the first amino acid and polymorphism detected by protein sequencing methods noted. C2L: chain 2 long (92 amino acids); C2S: chain 2 short (90 amino acids); C2ST chain 2 short truncated (80 amino acids). The presumed initiator methionine in the leader sequence is in bold type and the potential N-glycosylation site is underlined. (-) symbolizes complete agreement or identity with the amino acid residue listed above the (-).

Figure 8 shows the results of SDS/PAGE Western immunoblot analysis of affinity purified TRFP under reduced conditions probed with affinity purified rabbit anti-peptide antibodies (anti-Fel 2, Fel 4, and Fel 18); monoclonal anti-Fel d I antibody (6F9) and pooled cat allergic human serum IgE. The anti-Fel 2, Fel 4 antisera are specific for chain 1. The anti-Fel 18 antiserum is specific for chain 2.

Figure 9 is a graphic representation of the secondary T cell response of peripheral blood lymphocytes from patient 131 stimulated with various antigens and peptides.

As described herein, TRFP has been isolated and purified by affinity purification of vacuum cleaner bag house dust collected from several homes with cats. The work described herein has resulted in isolation and purification of a TRFP protein; determination of the nucleotide sequence encoding TRFP and the amino acid sequence of TRFP (Figures 1-7); demonstration that TRFP is composed of two covalently linked peptide chains (designated chain 1 and 2); identification and isolation of T cell reactive peptides or amino acid sequences present in the TRFP protein; and characterization of TRFP. It has also resulted in cloning and expression of TRFP in E. coli and characterization of the resulting recombinant TRFP proteins. As described in Example 4, cDNA clones encoding all or part of TRFP chain 1 or chain 2 have been expressed in E. coli as recombinant fusion proteins. Of note is the finding that chain 1 of the two-chain TRFP protein has two alternative leader sequences and that chain 2 has two major forms (designated as long and short).

A monoclonal antibody reactive with Felis domesticus allergen I, known as Fel d I, was used to isolate a single protein from a vacuum cleaner bag preparation. The affinity purified T cell reactive protein isolated in this manner is referred to as human T cell reactive feline protein (human TRFP). TRFP has been shown to have biological activity (human IgE binding ability) and to possess cross reactivity with rabbit anti-Fel d I antisera. The term "allergenic" as used herein in referring to peptides or proteins of the present invention refers to those peptides or proteins which bind IgE and/or stimulate T cells.

In addition to determining the amino acid sequence of chains 1 and 2 of the TRFP, a Fel d I protein preparation provided by Martin Chapman was analyzed and the protein was isolated and sequenced. Comparison of the amino acid sequence of the affinity purified TRFP with that of the published Fel d I protein sequence showed that there is a high degree of homology between the first 33 amino acid sequences at the amino terminus of Fel d I and chain 1 of TRFP.

The following is a description of the methods by which a single protein composed of two covalently linked chains was isolated from house dust, as well as a description of approaches used to identify and isolate DNA encoding the TRFP. Furthermore, a description of methods used to generate recombinant TRFP chains 1 and 2 are also presented. Additionally, human T cell epitopes from the TRFP protein have been identified and are described herein.

### Isolation of a single protein from a vacuum cleaner bag preparation

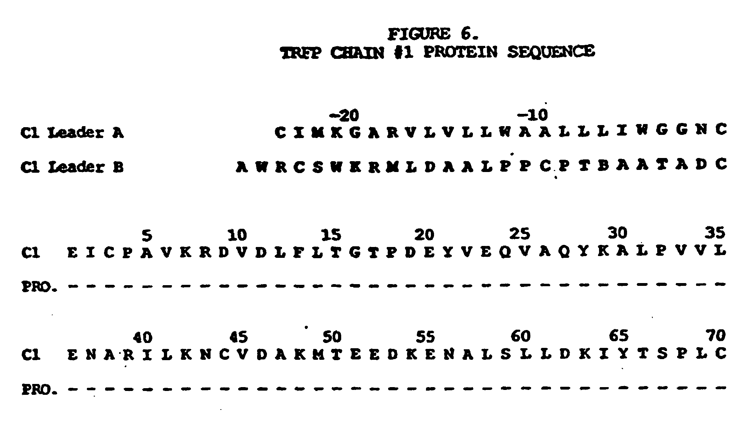

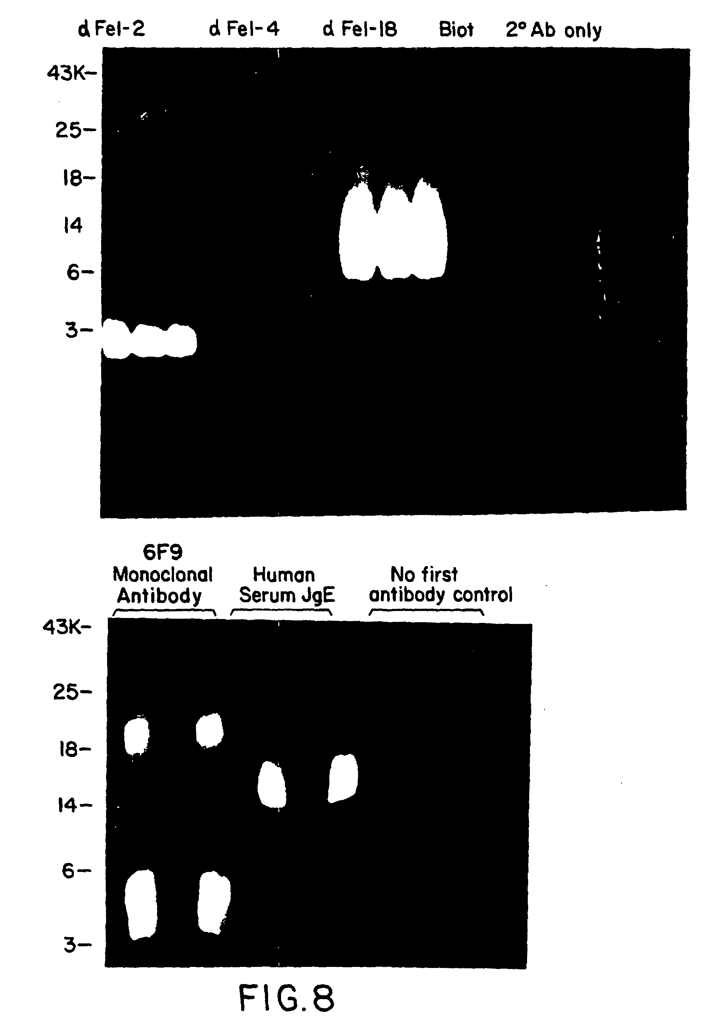

A protein preparation was extracted from the contents of vacuum cleaner bags by a method based on that of M.D. Chapman and co-workers. Chapman, M.D., et al, J. Immunol., 140(3): 812-818 (1988). Monoclonal antibody reactive with Fel d I, produced by Chapman and co-workers, was used to identify a protein in the preparation. de Groot H. et al., J. Allergy Clin. Immunol., 82:778-786 (1988). Selected monoclonal antibodies (designated 1G9 and 6F9) that recognize Fel d I native protein were used to affinity purify a protein, which is referred to as human T cell reactive feline protein(TRFP) (also referred to as VCB or vacuum cleaner bag protein) from a house dust sample. This was carried out, using known techniques, by producing the desired monoclonal antibody, isolating it in large quantities from ascites and immobilizing it on Sepharose 4B (Pharmacia). The protein preparation was extracted from vacuum cleaner bags of house dust obtained from several homes with cats. Aqueous vacuum cleaner bag extract was first subjected to gel filtration and decolorization and, subsequently, affinity chromatography purification. Aqueous vacuum cleaner bag extract was passed over the monoclonal antibody-containing column and a protein species was eluted. The protein isolated in this manner was shown, using both Western blot and ELISA techniques, to bind human IgE, thus demonstrating that TRFP possesses allergenic activity. The affinity purified TRFP was subjected to a number of protein chemical procedures to derive primary amino sequence data. The sequences derived from TRFP are illustrated in Figures 6 and 7. The methods used in the protein sequence analysis are further described in Example 1. Under non-reducing conditions, Western blot analysis demonstrated the existence of a 40kD and a 20kD species, whereas a 10-18 kD and a 5kD species was detected under reducing conditions (Figure 8).

The 5 kD band interacts with affinity purified anti-peptide antisera raised against peptides derived from chain 1 protein sequence (anti-Fel 2 and anti-Fel 4), whereas the 10-18 kD band interacts with antipeptide antiserum raised against peptide derived from chain 2 protein sequence (anti-Fel 18). Hence, the 5 kD band and the 10-18 kD band are derived from the TRFP chain 1 and the chain 2, respectively. TRFP can exist as an aggregated form, as demonstrated by the approximately 40 kD molecular weight of the affinity purified TRFP (may be a dimer of the chain 1 and chain 2 heterodimer) and the approximately 130 kD species detected in gel filtration prior to affinity purification.

### Identification of clones containing DNA inserts encoding the human T cell reactive feline protein (TRFP)

Protein chemical analysis of affinity purified TRFP led to the determination that TRFP is composed of two covalently linked peptide chains (designated chain 1 and 2; see Example 1 for details). Furthermore, peptide sequence analysis led to the determination of considerable primary sequence data for both chain 1 (70 amino acids; see Figure 6) and chain 2 (83 amino acids; see Figure 7). The amino acid sequence data was used to devise various cloning strategies to enable the cloning and complete nucleotide sequence determination of cDNAs and genomic clones encoding the TRFP chains 1 and 2 (details provided in Examples 2 and 3).

In order to determine the best tissue source(s) to isolate mRNA for the cloning of TRFP, various cat tissues were examined by ELISA techniques using monoclonal antibodies (directed against Fel d I). It was determined that the several salivary glands and skin contain significant levels of TRFP, and thus, provide a valuable source from which to clone cDNA sequences encoding the TRFP (see Table 1, in which --- indicates that an analysis was not done).

**Table 1**

| | Micrograms TRFP/gram tissue | | | | |
|---|---|---|---|---|---|
| Tissue | Cat 1 | Cat 2 | Cat 3 | Cat 4 | Cat 5 |
| Parotid | 1.03 | 1.41 | 0.81 | 0.32 | 0.30 |
| Mandibular | 0.41 | 2.39 | 7.50 | 2.50 | 4.66 |
| Sublingual | 0.77 | ---- | 0.50 | 3.18 | 3.82 |
| Zygomatic | ---- | ---- | 2.07 | 8.50 | 10.9 |
| Molar | ---- | ---- | 7.58 | 1.47 | 25.00 |
| Palate | ---- | ---- | 1.03 | 0.53 | 0.77 |
| Washed Skin | 5.80 | 2.30 | ---- | ---- | ---- |

Several approaches can be used to clone the TRFP encoding cDNAs including oligonucleotide screening of a λ gt10 or λ gt11 cDNA library. Alternatively, anti-peptide antisera reactive with TRFP amino acid sequences (see Figures 6 and 7) can be used to screen a gt11 library using standard methods. Young, R.A. and R.W. Davis, Proceedings of the National Academy of Sciences, USA, 80: 1194-1198 (1983). DNA can be isolated from reactive clones and sequenced using the method of Sanger and co-workers. Sanger, F. et al Proc. Natl. Acad. Sci., USA 74: 5463 (1977).

Another approach to clone DNA encoding the human T cell reactive feline protein is to use polymerase chain reaction (PCR) technology to amplify and clone DNA from cat salivary gland mRNA or genomic DNA. Mixed oligonucleotide primers (forward and reverse) were deduced from the known amino acid sequence and used in a PCR to produce a partial cDNA clone. This strategy, termed mixed oligonucleotide primed amplification of cDNA (MOPAC), is described by Lee and co-workers (Lee et al., Science, 239: 1288-1291 (1988)). MOPAC (and derived methods) have been used to isolate both partial and full-length cDNAs encoding the TRFP chains 1 and 2 (Detailed in Example 2 and the nucleotide sequences illustrated in Figures 1-6). The PCR derived cDNA clones were used as ³²P-labelled probes to screen a cat genomic EMBL4 library, as described in Example 3.

As a result of the work described herein, cDNAs and genomic clones encoding chain 1 and chain 2 of TRFP have been cloned, isolated and sequenced; the encoded amino acid sequences of the protein has been deduced; and peptides derived from TRFP have been identified and isolated using known methods. The complete nucleotide sequences encoding both TRFP chains are shown in Figures 1-5. The hybridization pattern of individual genomic clones verified that the chain 1 and chain 2 cDNAs are products of different genes. Northern blot analysis of the cat salivary gland RNA also demonstrated the presence of the two separate mRNAs. Sequencing of the genomic clones confirmed the hybridization results. As described in Example 2, individual full-length PCR generated chain 1 clones were shown to have two different sequences at their 5' ends, suggesting that chain 1 has two alternative leader sequences. This was confirmed by the DNA sequence analysis of the chain 1 genomic clone, which demonstrated that the single chain 1 gene has both alternative leader sequences closely linked at the 5' end of the structural gene (see Figures 1, 2 and 6).

As described in Example 4, cDNA clones encoding all or a fragment of TRFP chain 1 or chain 2 were subcloned into E. coli expression vectors and the expressed recombinant TRFP proteins examined. Western blot analysis using rabbit anti-peptide antisera directed against either chain 1 sequences or chain 2 sequences demonstrated appropriate binding specificity.

### Uses of the subject human T cell reactive feline protein (TRFP) and DNA encoding same

The materials resulting from the work described herein, as well as compositions containing these materials, can be used in methods of diagnosing, treating and preventing cat allergy. In addition, the cDNA (or the mRNA from which it was reverse transcribed) can be used to identify similar sequences in other species (e.g., sheep, goat, dog, rabbit, horse) and, thus, to identify or "pull out" sequences that have sufficient homology to hybridize to the TRFP cDNA. Such sequences from other species might encode proteins useful in treating allergies to these animals in people. This can be carried out, for example, under conditions of low stringency and those sequences having sufficient homology (generally greater than 40%) can be selected for further assessment using the method described herein. Alternatively, high stringency conditions can be used. In this manner, DNA of the present invention can be used to identify, in other types of mammals (e.g., dog, rabbit, sheep, goat, horse), sequences encoding peptides having amino acid sequences similar to that of the TRFP. This can be done by hybridization or PCR cloning methods. Thus, the present invention includes not only the TRFP or peptide encoded by the present DNA sequences, but also other TRFP-like proteins or allergenic peptides encoded by DNA which hybridizes to DNA of the present invention.

The TRFP peptide encoded by the cDNA of the present invention can be used, for example, as "purified" TRFP, in a composition to treat cat-allergic individuals, in a method to diagnose cat allergy, or in the standardization of allergen extracts which are key reagents for the diagnosis and treatment of cat allergy. Through use of the protein of the present invention, allergen preparations of consistent, well-defined composition and biological activity can be made and administered for therapeutic purposes (e.g., to modify the allergic response of a cat-sensitive individual to cat allergies). Such a protein or peptide (or modified version thereof, such as is described below) may, for example, modify B-cell response to cat allergen, T-cell response to cat allergen or both responses. Purified TRFP or peptide thereof can also be used to study the mechanism of immunotherapy of cat allergy and to design modified derivatives or analogues that are more useful in immunotherapy than are the unmodified ("naturally-occurring") protein or peptide.

Work by others has shown that high doses of allergens during immunotherapy treatment generally produce the best results (i.e., best symptom relief). However, many people are unable to tolerate large doses of allergens because of adverse reactions to the allergens.

The present invention enables the production of therapeutic treatments for cat allergic individuals which will possess similar or improved efficacy to that of current allergen immunotherapy without the adverse reactions normally associated with this form of therapy. Improved therapy could derive from use of modified naturally occurring TRFP or peptide expression products of the TRFP genes identified herein or appropriate modifications (mutations) thereof, or peptides derived from the structure of TRFP or modifications thereof.

For example, the naturally occurring TRFP or peptide can be modified using the polyethylene glycol method of A. Sehon and coworkers or in other ways which reduce the IgE reactivity of the natural allergen and thereby decrease its adverse reaction potential.

Alternatively, the TRFP cDNAs defined herein, or portions thereof, can be expressed in appropriate systems to produce protein(s) with strong therapeutic activity, but greatly reduced ability to bind to IgE and thereby produce adverse reactions. To facilitate this, it is possible to add reporter group(s) to the chain 1 and/or 2 polypeptide backbone as an aid to efficient purification. One such reporter group is poly-histidine, which has been effectively used to purify recombinant proteins on immobilized metal ion affinity chromatography (Hochuli, E. et al., Bio/Technology 6:1321-1325 (1988)). Specific cleavage sites can be introduced between the reporter group and the chain 1 and 2 polypeptide sequences, and cleavage at these sites can facilitate the isolation of TRFP chains or fragments free of irrelevant sequences. Another example of the modification of the TRFP chains 1 and 2 is the substitution of cysteine residues with another amino acid residue such as serine (or any other residue) to reduce disulfide complexes.

Site-directed mutagenesis of the TRFP cDNAs can also be used to modify the chain 1 and 2 structures. Such methods may involve PCR (Ho et al., Gene 77:51-59 (1989)) or total synthesis of mutated genes (Hostomsky, Z. et al., Biochem. Biophys. Res. Comm. 161:1056-1063 (1989)) since the two chains are each composed of coding sequences <400bp. To enhance bacterial expression, the aforementioned methods can be used in conjunction with other procedures to change the mammalian codons in the constructs to ones preferentially used in E. coli.

Other modifications of the TRFP genes may include the construction of gene chimeras, where chains 1 and 2, or parts thereof, may be linked to form a single contiguous chain. For example, all or a portion of chain 1 may be linked with all or a portion of chain 2 cDNA and the resulting chimera may be produced as a recombinant hybrid (Horton et al., Gene 77:61-68 (1989)). It is also possible to construct multiple joined genes to promote stability of the expressed product or to enhance its therapeutic potential (Shen et al., Proc. Natl. Acad. Sci. USA 81:4627-4631 (1984)).

The work described herein has further resulted in identification of certain areas of the TRFP protein which contain peptide epitope sequences which powerfully stimulate T cells from cat allergic individuals. It is believed that these T cell epitopes are intimately involved in initiation and perpetuation of the immune responses to cat allergen(s) which are responsible for the clinical symptoms of cat allergy. Such epitopes from the natural cat allergen(s) are believed to trigger early events in the allergic cascade at the level of the T helper cell by binding to an appropriate HLA molecule on the surface of an antigen presenting cell and stimulating the relevant T cell subpopulation. These events lead to T cell proliferation, lymphokine secretion, local inflammatory reactions and the recruitment of additional immune cells to the site and activation of the B cell cascade leading to production of antibodies. One isotype of these antibodies, IgE, is fundamentally important to the development of allergic symptoms and its production is influenced early in the cascade of events, at the level of the T helper cell, by the nature of the lymphokines secreted. Exogenously administered T cell epitope peptides may thus influence the development or perpetuation of the allergic response to cat allergens and produce therapeutic benefit in cat allergic individuals. Thus, exposure of cat allergic patients to T cell epitope peptides identified as described herein may tolerize or anergize appropriate T cell subpopulations so that they no longer respond to cat allergen(s) and do not participate in mounting the immune response to such exposure. Alternatively, administration of the T cell epitope peptides may drive the lymphokine secretion profile in a different direction than is the case with exposure to natural allergen(s) (e.g. decreased IL-4 and/or increased IL-2), resulting in a reduction of local inflammatory events and/or a beneficial change in the antibody secretion profile. Alternatively, exposure to T cell epitope peptides may cause T cell subpopulations which normally participate in the response to cat allergen(s) to be drawn away from the site(s) of normal exposure to the allergen (nasal mucosa, skin, lung) towards the site(s) of therapeutic administration of the peptides. This redistribution of T cell subpopulations could ameliorate or reduce the ability of an individual's immune system to mount the usual immune response to the allergen(s) at the site of normal exposure, leading to a diminution in allergic symptoms.

Peptides of varying sizes from within the structure of TRFP have been synthesized and purified by conventional techniques and examined for their biological effects on T cell lines and clones obtained from human cat allergic individuals. The methodologies used are described in Examples 5, 6 and 7.

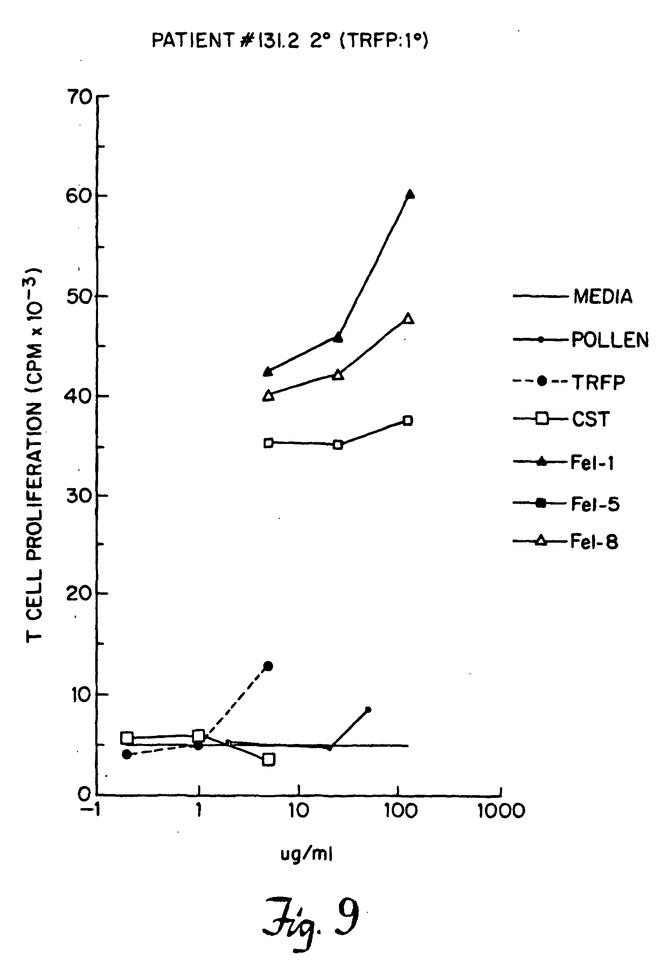

Peptides from within the structure of TRFP have been shown to stimulate a proliferative response in TRFP primed T cell cultures. Indeed in certain cases (Figure 9), the proliferative response obtained with the peptides can substantially exceed that obtainable with TRFP or cat skin test allergen preparations.

It is also apparent (Tables 2 and 3) that certain areas of the TRFP sequence have weak T cell stimulatory activity (e.g. Fel 4-3, Fel 28-1); certain other areas have powerful activity (e.g. Fel 8-3, Fel 14). This range of activities may derive from purely primary sequence differences or from physicochemical differences induced by primary sequence changes. Key peptide epitopes from within the structure of TRFP which are highly reactive with T cells from cat allergic patients can be identified using the subject disclosure and known techniques.

Furthermore, it has been demonstrated that exposure of T cells to epitope peptides in vitro under conditions which simulate therapeutic treatment regimens can suppress a subsequent response to the allergen (TRFP) to a greater extent than that obtained with the allergen alone (Example 6, Table 4). This data points to the clear opportunity of selecting epitope peptides identified by the current work and applying them in treatment paradigms in cat allergic patients designed to suppress their response to cat allergen exposure.

In addition, it has been shown that exposure of T cells from cat allergic individuals to different epitope peptides from TRFP can produce distinctly different lymphokine secretion profiles (Example 7, Table 5). It is thus possible using the current invention to select for therapeutic application epitope peptides which drive a lymphokine secretion profile consistent with a therapeutically beneficial response upon treatment of cat allergic patients.

Administration of a TRFP protein or peptide of the present invention, which can be substantially pure TRFP, recombinant TRFP, modified TRFP, alone or in combination, to an individual to be desensitized can be carried out using known techniques. A peptide or combination of two or more different peptides can be administered to an individual in a composition which also includes, for example, an appropriate buffer, a carrier and/or an adjuvant. Such compositions will generally be administered by injection, inhalation, transdermal application, intranasal application, oral application or rectal administration. Using the structural information now available, it is possible to design a TRFP or peptide that, when administered to a cat-allergic individual in sufficient quantities, will modify the individual's allergic response to cat allergen. This can be done, for example, by examining the structures of the TRFP, producing peptides to be examined for their ability to influence B-cell and/or T-cell responses in cat-allergic individuals and selecting appropriate epitopes recognized by the cells. Synthetic amino acid sequences which mimic those of the epitopes and which are capable of down regulating allergic responses to cat allergen can be produced. The protein, peptide or antibodies of the present invention can also be used, in known methods, for detecting and diagnosing cat allergy. For example, blood obtained from an individual to be assessed for sensitivity to cat allergen is combined with an isolated peptide of TRFP, under conditions appropriate for binding of components (e.g., antibodies, T cells, B cells) in the blood with the peptide. Subsequently, the extent to which such binding occurs is determined, using direct (e.g., determination of T cell activation) or indirect methods.

It is now also possible to design an agent or a drug capable of blocking or inhibiting the ability of cat allergens to induce an allergic reaction in cat-allergic individuals. Such agents can be designed, for example, in such a manner that they would bind to relevant anti-cat allergen IgEs, thus preventing IgE-allergen binding and subsequent mast cell degranulation. Alternatively, it is possible to design agents which bind to cellular components of the immune system, resulting in suppression or desensitization of the allergic response to cat allergen. A non-restrictive example of this is the use of appropriate B- and T-cell epitope peptides, or modifications thereof, based on the cDNA/protein structure of the human T cell reactive feline protein of the present invention to suppress the allergic response to cat allergen. This can be carried out by defining the structures of B- and T-cell epitope peptides which affect B- and T-cell function in in vitro studies with blood cells from cat-sensitive individuals. This procedure is described in detail in Example 5.

It is also possible to modify epitopes of the TRFP, to combine epitopes, or to do both, for such purposes as enhancing therapeutic or preventive efficacy, stability (e.g., length of time for which they can be stored), and resistance to degradation in the body of TRFP peptides. For example, the amino acid residues essential to epitope function can be determined using known techniques (e.g., substitution of each residue and determination of presence or absence of T cell reactivity). Those residues shown to be essential can be modified (e.g., replaced by another amino acid whose presence is shown to enhance T cell reactivity), as can those which are not required for T cell reactivity (e.g., by being replaced by another amino acid whose incorporation enhances T cell reactivity).

Two or more TRFP epitopes can also be combined in order to enhance, for example, therapeutic effectiveness. For example, the amino acid sequences of two epitopes present within the first 30 N-terminal amino acids can be produced and joined by a linker. The linker by which the epitopes are joined can be any non-epitope amino acid sequence or other appropriate linking or joining agent. The epitopes joined in this manner can be from the same chain of the TRFP or from different TRFP chains (e.g., one from chain 1 and one from chain 2). The resulting two-epitope construct can be used in treating cat-sensitive individuals. Alternatively, an epitope (or epitopes) present in the first chain of the TRFP and one (or more) present in the second chain can be joined to produce a construct which has greater therapeutic effectiveness than a single epitope peptide. Additionally, individual peptides can be physically mixed and administered as a therapeutant.

DNA to be used in any embodiment of this invention can be cDNA obtained as described herein or, alternatively, can be any oligodeoxynucleotide sequence that codes for all or a portion of the amino acid sequence represented in Figures 1-7, or the functional equivalent thereof. Such oligodeoxynucleotide sequences can be produced chemically or mechanically, using known techniques. A functional equivalent of an oligonucleotide sequence is one that is capable of hybridizing to a complementary oligonucleotide sequence to which the sequence (or corresponding sequence portions) of Figures 1-7 hybridizes and/or that encodes a product (e.g., a polypeptide or peptide) having the same functional characteristics of the product encoded by the sequence (or corresponding sequence portion) of Figures 1-7. Whether a functional equivalent must meet one or both criteria will depend on its use (e.g., if it is to be used only as an oligoprobe, it need meet only the first criterion and if it is to be used to produce an allergen, it need meet only the second criterion).

The structural information that is available or can be deduced from the amino acid sequences of Figures 1-7 (e.g., DNA, protein/peptide sequences), can also be used to identify or define T cell epitope peptides and/or B cell epitope peptides which are of importance in cat allergic reactions and to elucidate the mediators or mechanisms (e.g., interleukin-2, interleukin-4, gamma interferon) by which these reactions occur. This knowledge should make it possible to design peptide-based cat allergen therapeutic agents or drugs which can be used to modulate these responses.

The present invention will now be further illustrated by the following Examples, which are not intended to be limiting in any way.

### EXAMPLE 1. Isolation and Protein Sequence Analysis of the T Cell Reactive Feline Protein (TRFP)

### Monoclonal affinity purification of a T cell reactive feline protein from house dust extract

A house dust sample collected from several homes with cats was used to isolate and purify TRFP. Monoclonal antibodies 1G9 or 6F9 were coupled to Sepharose 4B and used for the purification according to a published protocol. Chapman, M.D., et al., J. Immunology, 140(3):812-818, (1988). The purified TRFP was decolorized by loading it on a Phenyl-Sepharose column (Pharmacia) with 4N NaCl, then eluted with 2M and 1M NaCl. Decolorized TRFP was recovered by dialyzing the 2M and 1M salt eluates against distilled water and lyophilized. Decolorization was also carried out by passing the house dust extract through a Sephacryl 200 column (Pharmacia) before the affinity purification.

### Preparation of TRFP peptides

Affinity purified TRFP was first reduced with dithiothreitol and then alkylated with 4-vinyl pyridine. After desalting with a Sephadex G10 column, the reduced and alkylated TRFP was cleaved chemically with cyanogen bromide (CNBr) or enzymatically with one of the following enzymes: endoproteinase Glu-C (Boehringer Mannheim), endoproteinase Asp-N (Boehringer Mannheim), endoproteinase Lys-C (Boehringer Mannheim). The affinity purified TRFP was also digested by trypsin (Worthington) without reduction and alkylation. The digestion products were separated on an Aquaport RP300 column (C8) with acetonitrile gradient in 0.1% trifluoroacetic acid (TFA). The individual peaks were subjected to the protein sequencer.

### Peptide and protein sequence analysis

An Applied Biosystems Model 477A gas phase sequencer with on-line phenylthiohydantoin (PTH) amino acid analysis (Model 120A) was used. A modification of extraction program, multiple butylchloride extractions, was used to improve the amino acid recovery. O-phthalaldehyde was used in blocking of primary amines when proline was located at the amino terminus. Brauer, A.W., et al., Anal. Biochemistry, 137:134-142, (1984). In situ alkylation was performed by using the non-nucleophilic reductant, tributylphosphine with concomitant alkylation by 4-vinyl pyridine in ethylmorpholine buffer. Andrews, P.C. and Dixon, J.E., Anal. Biochemistry, 161:524-528 (1987). The N-terminal protein sequence analysis of the intact TRFP protein revealed that there is one major amino acid sequence and several minor amino acid sequences. The major sequence (chain 1 in Figure 6) corresponds to the published Fel d I N-terminal 33 amino acid residues with two significant differences. Chapman, M.D., et al., J. Immunology, 140(3):812-818, (1988). The most prevalent minor sequence (at 55% the level of the major sequence) was designated as chain 2 (Figure 7). The other minor sequences were various N-terminal truncated forms of chain 2. Since the 4th residue of chain 1 and the 7th residue of chain 2 were proline, o-phthaladehyde was applied before the 4th cycle or the 7th cycle of Edman degradation to block out chain 2 or chain 1 sequences, respectively. The protein sequence information of chain 1 (68 N-terminal amino acid residues) and chain 2 (58 N-terminal amino acid residues) was further enhanced by an additional OPA blocking before cycle 32 and cycle 37 for chain 1 and chain 2, respectively. The protein sequences were confirmed and expanded by sequence analysis of enzymic and chemical digested peptides. Time dependent in situ CNBr digestion of TRFP on the sequencer glass filter disk could provide additional protein sequence information. Simpson, R. J. and Nice, E. C., Biochem. International, 8:787-791 (1984). Prior to the in situ CNBr digestion five sequencer cycles were performed and then the protein sample was treated with acetic anhydride to block all amino groups. These steps removed the N-terminal five residues from both chains and blocked the amino groups of the next residue from both chains and any other peptide in the sample. After 5 hours of in situ CNBr digestion, one major peptide sequence, CB-1, and three minor peptides which had 60% (CB-2), 38% (CB-3), and 12% (CB-4) signal levels of the major peptide sequence were identified. CB-1 started from residue 43 of chain 2 and extended the N-terminal sequence of chain 2 to 68 residues. CB-2 was identical to a purified CNBr peptide sequence of chain 2 (75-80). CB-3 corresponded to the peptide sequence 65-68 of short form chain 2. CB-4 corresponded to the peptide sequence 50-66 of chain 1. A tryptic peptide TRYP-1 (short form chain 2, 58-80) connected the 68 residue N-terminal peptide with an endopeptidase Asp-N generated peptide, D-10 (chain 2, 72-83), and extended the chain 2 to 83 amino acid residues. An endopeptidase Lys-C generated peptide, K13 (chain 1, 64-70), extended chain 1 to 70 amino acid residues. Other enzymic and CNBr generated peptides confirmed the N-terminal sequences of chain 1 and chain 2. The sequences of a short tryptic peptide, TRYP-2 (long form chain 2, 58-69), and an endopeptidase Asp-N peptide, D-10, revealed that there was sequence polymorphism in chain 2 residues 65 to 72. In summary, the primary amino acid sequence of TRFP chains 1 and 2 derived by protein sequencing methods is presented in Figures 6 and 7.

There is a potential N-glycosylation site present in the cDNA deduced amino acid sequence, Asn₃₃-Ala₃₄-Thr₃₅. The protein sequence analysis identifies the Ala₃₄ and Thr₃₅ of chain 2; however, nothing can be identified at the position 33. It suggests that post-translation modification occurs at Asn₃₅ of chain 2 and the modification is stable to the trifluro acetic acid treatment during protein sequencing. The hypothesis was confirmed by treating TRFP with N-peptidase F (Boehringer Mannheim) which reduced the size of chain 2 to 7-12 kD in SDS-PAGE/Western immunoblot. Moreover, both chains can be modified by β-elimination which implies they may have O-linked glycosylation. The two chains are covalently linked together (approximately 20kD) through disulfide bond(s).

### EXAMPLE 2. Cloning of cDNAs Encoding Chains 1 and 2 of the Human T cell Reactive Feline Protein (TRFP)

MOPAC (and derived methods) have been used to isolate both partial and full-lengthcDNAs encoding the TRFP chains 1 and 2. The PCR methods used are described in detail below.

### Cloning of TRFP chain 1 cDNAs

First strand cDNA synthesis was performed with 1 µg of poly A plus RNA isolated from a pooled sample of cat parotid and mandibular glands using oligo dT primer.

MOPAC PCR amplification (Lee et al, Science 239:1288-1291(1988)) of an internal portion of chain 1 was carried out using a sense/antisense pair of degenerate oligonucleotide primers encoding amino acids 1-6 and 50-54 of chain 1, respectively (see below). These oligonucleotides (primers 1 and 2) were used with a Perkin Elmer/Cetus PCR kit to amplify an aliquot of the above cDNA using the following cycles:

| | | |
|---|---|---|
| 94° C 1 min. | (denaturation) | |
| 45° C 1 min. 30 sec. | (annealing) | |
| 72° C 1 min. | (polymerization) | 5 Cycles |
| 94° C 1 min. | (denaturation) | |
| 55° C 1 min. 30 sec. | (annealing | |
| 72° C 1 min. | (polymerization) | 20 Cycles |

One tenth of the above PCR reaction was fractionated on a 3% NuSieve Agarose gel. A DNA band of the predicted size (172 base pairs) was observed. This gel was then "Southern" blotted onto GeneScreen Plus nylon membrane under denaturing conditions and hybridized to ³²P end-labeled chain 1 specific oligonucleotide probe (Fel 1) in 6 X SSC at 35 degrees C, and washed in 2 X SSC at 48 degrees C. The 173 base pair band hybridized to the chain 1 specific probe.

The remainder of the PCR reaction was restriction digested with Cla I and Xho I and fractionated over a preparative 3% NuSieve agarose gel and the 173 base pair band excised. The fragment was ligated into Cla I/Xho I digested Bluescript plasmid (Stratagene), and subjected to Sanger/dideoxy DNA sequence analysis using a Sequenase kit (US Biochemicals). The data from this analysis shown in Figure 1 demonstrated that the sequence of the PCR amplified DNA fragment, when translated, is in agreement with an internal portion of the protein sequence of chain 1 of TRFP.

The 3' end of the chain 1 cDNA encoding TRFP was cloned according to the RACE PCR method. Frohman, M.A., Dush, M.K., and Martin, G.R. Science 85: 8998-9002. (1988).

First strand cDNA synthesis was performed with 1 µg of poly A plus RNA isolated from a pooled sample of cat parotid and mandibular glands using the EDT primer with Superscript reverse transcriptase.

RACE PCR amplification of the carboxy terminal portion of chain 1 was carried out using primer 3 and the ED primer as the 5' and 3' specific primers respectively. Primers were used with a Perkin Elmer/Cetus PCR kit to amplify an aliquot of the above cDNA using the following cycle:

| | | |
|---|---|---|
| 94° C 1 min. | (Denaturation) | |
| 55° C 1 min. 30 sec. | (Annealing) | |
| 72° C 1 min. | (polymerization) | 30 Cycles |

One tenth of the above PCR reaction was fractionated on a 2% agarose gel. After "Southern" blotting of the gel onto GeneScreen Plus nylon membrane and hybridization to a chain 1 specific oligonucleotide probe (Fel 1), as above, no bands that could be candidates for cDNAs encoding the 3' portion of the TRFP Chain 1 were detected.

A second PCR reaction with cycling identical to that used for the first amplification was performed with a 1/100th aliquot of the initial PCR reaction products as template and primer 4 (encoding amino acids just 3' of those encoded in primer 3) and the ED oligonucleotide as primers. This "nested" PCR rection served to specifically reamplify products from the primary PCR reaction derived from TRFP chain 1 cDNA.

One tenth of this second PCR reaction was fractionated on a 2% agarose gel. After "Southern" blotting of the gel onto GeneScreen Plus nylon and hybridization to a chain 1 specific oligonucleotide probe, as above, a DNA band about 350 base pairs in length was detected.

The remainder of the second PCR reaction was restriction digested with Cla I and Xba I, and fractionated over a preparative 1% SeaPlaque agarose gel and the 350 base pair band excised. The fragment was ligated into Cla I/Xba I digested Bluescript plasmid (Stratagene), and subjected to Sanger/dideoxy DNA sequence analysis using a Sequenase kit (US Biochemicals). The data from this analysis shown in Figure 1 demonstrates the sequence of PCR amplified 350 base pair DNA fragment, when translated, is in agreement with the protein sequence at the carboxy terminus of chain 1 of TRFP. The DNA sequence analysis also reveals a stop codon adjacent to the cysteine codon at position 72, indicating the protein sequence analysis of chain 1 of TRFP had been done in its entirety. In addition, 3' untranslated DNA sequence of the 350 base pair fragment contains a prototypical polyadenylation signal characteristic of the 3' end of a cDNA.

### Cloning of TRFP chain 2 cDNAs

First strand CDNA was synthesized with a commercial kit using oligo (dT) priming of mRNA prepared from a pool of the parotid/mandibular glands of five cats. An internal sequence of chain 2 was determined using MOPAC.

Two redundant oligomers were synthesized based on protein sequence of human T cell reactive feline protein Chain 2: 
                                              G——
                          G         G  A    T
Oligomer #56.   5'  CGGAATTCAA ATGGCIGA AC TGY C,
                          A         A  T    C
                                       C
 which coresponded to coding strand sequence encoding amino acids 2-8 (KMAETCP) and 
                                 G  G  T     T  T
Oligomer #57.    5'  CGGGCTGCAGTA CA TC TGTAT TT TTCAT,
                                 A  A  C     C  C
 which corresponded to non-coding strand sequence complimentary to amino acids 42-48 (MKKIQDCY) Oligomer 56 bad an Eco RI restriction site added (underlined) and oligomer 57 had a Pst I restriction site added (underlined) for cloning purposes. Inosine (I) was used once in each oligomer to reduce the redundancy of the final oligomers as described in Knoth et al. 1988. Nucl. Acids Res. 16:10932.

PCR was performed using 100 pmol of each primer plus first strand cDNA using the following conditions for amplification:

Denature at 94 degrees C for 1 min.; primer anneal at 45 degrees C for 1.5 min.; elongate at 72 degrees C for 2 min.; repeat cycle 4 times.

Denature as above; anneal at 55 degrees C for 1.5 min.; elongate as above; repeat second cycle 19 times (total of 25 cycles).

Two cDNA clones containing Chain 2 sequence were identified. Both clones had identical sequence. The prototype clone is F2.m.

The COOH end of the chain 2 cDNA encoding TRFP was cloned according to the RACE PCR method. First strand cDNA was synthesized from mRNA as described above.

Oligomers used in amplification of Chain 2 were: Oligomer #59. 5' GGATCGATGAATTCGGTGGCCAATGGAAATG, which corresponded to coding strand sequence encoding amino acids 19-23 (VANGN) of Chain 2 and contained Cla I and Eco RI restriction sites for cloning purposes. Oligomer #61. 5' ATTACTGTTGGACTTGTCCCT, which corresponded to amino acids 23-28 (LLLDLS) of Chain 2, and ED/EDT primers described above.

Two PCR reactions were carried out using "nested primers." The primary PCR reaction used 100 pmol of oligomer 59 and 100 pmol of the ED and EDT primers in a 3:1 ratio. Amplification conditions were the same as those used in obtaining internal Chain 2 sequence. 0.01 volume of the primer PCR was reamplified using 100 pmol of oligo #61 (a "nested" primer) and 100 pmol of the ED primer using the standard conditions.

The amplified fragment was cloned and sequenced to give the COOH end of Chain 2. There are two prototype clones: F15.a and F15.d. F15.a matched one protein sequence of the dominant protein sequence for Chain 2 while F15.d matched a second protein sequence. F15.a has been called the "Long" sequence and F15.d has been called the "Short" sequence. There are 7 clustered amino acid differences between F15.a and F15.d including 5 amino acid changes and two amino acid deletions in F15.d relative to F15.a (see Figures 6 and 7).

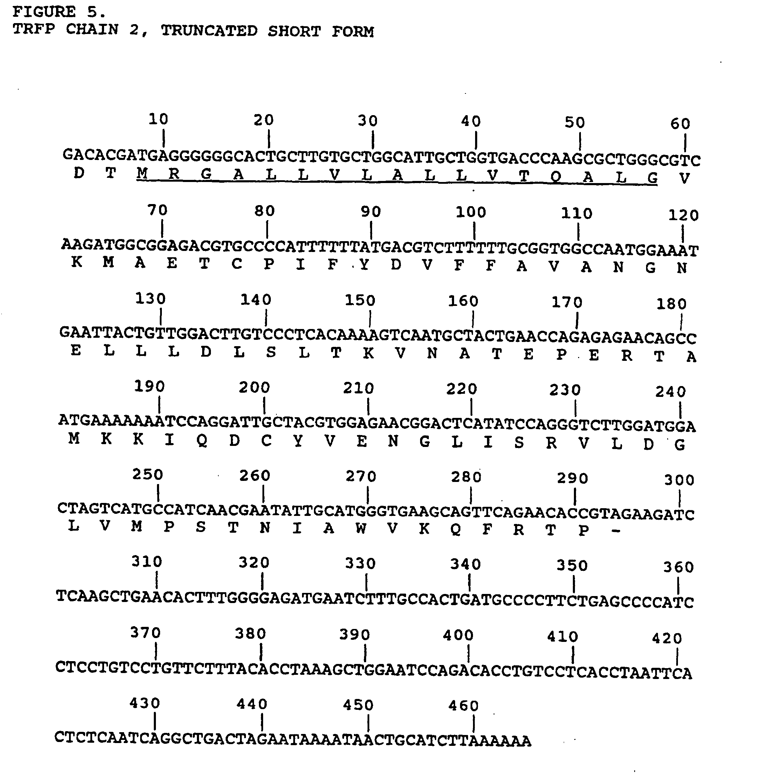

Chain 2 has been isolated from the mRNA from two cat skins as well as from mRNA from pooled salivary glands. The skin samples were sampled separately. Skin A (one of the five cats used in making the salivary gland pool) had mRNA encoding only the short form of Chain 2. Skin B (from a sixth cat not part of the five used in making the salivary gland pool) contained mRNA for both the Short and Long forms of Chain 2 in a 3:1 (S:L) ratio. A third form of Chain 2 has been found in the skin. This is called "ST" for Short Truncated (Figure 5). ST has 16 contiguous amino acid differences from the short form and has deleted the last 10 amino acids of the Short sequence. Examples of this clone have been found in mRNA from both Skin A and Skin B. Chain 2 Long is the dominant form of Chain 2 in the salivary glands (23/24 clones). Chain 2 Short is the dominant form of Chain 2 in the skin (20/25 clones from two cats), while the Long form (0/13 clones in Skin A and 3/12 clones in skin B) and the ST form (2/25 clones from two cats) appear to be minor forms. A summation of the complete nucleotide sequence of TRFP chains 1 and 2 derived by the methods cited above is presented in Figures 1-5.

Polymorphism in the long form of chain 2 was detected in the skin mRNA from one cat. This polymorphism involved the substitution of a Leucine for Isoleucine at amino acid 55 and a Threonine for a Methionine at position 74.

### Cloning the NH₂ terminals of TRFP chain 1 and chain 2

First and second strand cDNA was synthesized with a commercial kit using oligo dT priming of mRNA prepared from a pool of the parotid/mandibular glands of five cats. The double-stranded cDNA was blunted and then blunt-end ligated to annealed oligomers #68 and #69 (see below). These oligomers, described in Rafnar et. al., J. Biol. Chem., in press, were designed to utilize the "Anchored PCR" as described by Frohman et. al. 1988. Proc. Natl. Acad. Sci. USA 85:8998-9002 and modified by Roux and Dhanarajan. 1990 BioTech. 8:48-57. These oligonucleotides are not entirely homologous and, thus, will not self-prime. The oligomers will blunt end to every cDNA.

Oligomer #68. Template, Blunt Anchor
5' GGGTCTAGAGGTACCGTCCGATCGATCATT

Oligomer #69. Linker, Blunt Anchor
5' p-AATGATCGATGCT

Oligomer #64 Anchor Primer (AP) was
5' GGGTCTAGAGGTACCGTCCG

### Cloning the NH₂ terminal of TRFP Chain 2

Two oligomers based on internal chain 2 nucleotide sequence were synthesized: Oligomer #60. 5' CGGGCTCGAGCTGCAGCTGTTCTCTCTGGTTCAGT, which corresponded to non-coding strand sequence complementary to that encoding amino acids 35-40 (TEPERT), and Oligomer #70. 5' GGGCTGCAGATTCTAGTCAGCCTGATTGA, which corresponded to non-coding strand sequence of the 3' UT region. Both oligomers matched the antisense strand sequence and contained Pst I and Xho I restriction sites (underlined, Oligomer 60) or Pst I (underlined, Oligomer 70) for cloning purposes.

Two PCR reactions were carried out using "nested primers". Amplification conditions were the same as those used for MOPAC. The 1° amplication reaction was done with oligomers #64 (AP) and #70. 0.01 vol of the 1° PCR product was reamplified with oligomers #64 and #60 (which is "nested," i.e. internal, relative to oligo #70). Amplified material was recovered, cloned and sequenced.

### Cloning the NH₂ terminal of TRFP chain 1

One oligomer based on internal chain 1 sequence was synthesized: Oligomer #66, 5' GGGCTCGAGCTGCAGTTCTTCAGTATTCTGGCA, corresponded to non-coding strand sequence complementary to that encoding amino acids 38-43 (ARILKN) of chain 1. The restriction sites Pst I and Xho I were added for cloning purposes.

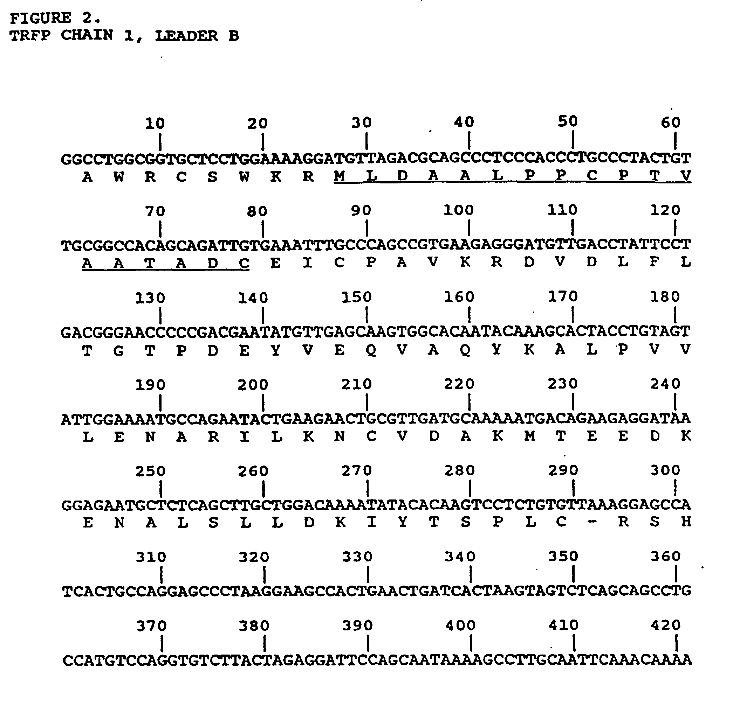

Two PCR reactions were carried out using "nested primers". Amplification conditions were the same as those used for MOPAC. 1° PCR was performed with primer 2 (described above) and #64 (AP). 0.01 vol of the 1° PCR product was reamplified with oligomers #64 and #66 (which is "nested," i.e. internal, relative to primer 2). Amplified material was recovered from the 2° PCR, cloned and sequenced. Two different 5' sequences were obtained and designated Leaders A and B (Figures 1 and 2).

Chain 1 with Leader A is a dominant sequence in both salivary gland and skin mRNA. It was not possible to detect chain 1 with Leader B sequence in the mRNA preparation from Skin A. Chain 1 with Leader B sequence was a minor component of the mRNA in both the pooled salivary gland and Skin B preparations.

### EXAMPLE 3. Screening of a Cat Genomic DNA Library to Identify Clones Containing DNA Encoding the TRFP

An EMBL4 cat genomic library, using cat liver DNA as starting material, was constructed using recommended procedures described in Frischauf, A.-M. et al. J. Mol. Biol. 170:827-842 (1983). The EMBL4 cat genomic library was screened using ³²P-radiolabelled chain 1 and chain 2 cDNA as probes. The library was plated out and screened, yielding individual genomic clones that hybridized to either chain 1 or chain 2 cDNA probes, but not to both. This hybridization pattern verified that the chain 1 and chain 2 cDNAs are products of different genes. Northern blot analysis of the cat salivary gland RNA probed with ³²P-radiolabelled TRFP chain 1 or 2 cDNA also demonstated the presence of the two separate mRNAs. The DNA sequence of the genomic clones (designated CTGch1 and CTGch2) was determined and confirmed the hybridization results.

Individual full length PCR generated chain 1 clones (Example 2) were shown to have two different sequences at their 5' end (see Figures 1 and 2). One interpretation is that chain 1 has two alternative leader sequences. The DNA sequence of the chain 1 genomic clone (CTGch1) has confirmed this interpretation and demonstrated that the single chain 1 gene possesses both alternative sequences closely-linked at the 5' end of the structural gene.

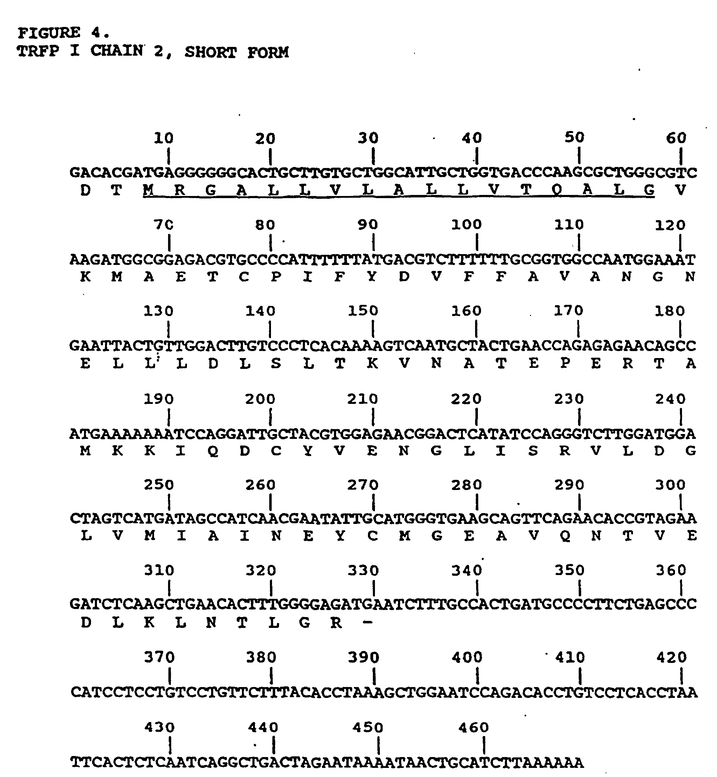

The DNA sequence of the chain 2 genomic clone (CTGch2) demonstrated the presence in the cat genome of different gene segments encoding the long and short forms of chain 2 (see Figures 3 and 4). The isolation of two genes encoding the TRFP chain 2 is consistent with the tissue specific expression of the two different mRNA forms in cat skin and salivary gland (Figures 3, 4 and 7; see Example 2).

Of note is that a comparison of the genomic sequences to that of isolated cDNAs demonstrated that the TRFP has sequence microheterogeneity.

### EXAMPLE 4. Expression of Recombinant TRFP chains 1 and 2

cDNA clones encoding all or parts of TRFP chain 1 or chain 2 have been subcloned into E.coli expression vectors, specifically pSEM-1, -2 and -3 (Knapp, S. Broker, M. and E. Amann. BioTechniques 8: 280-281. (1990). These vectors carry a truncated form of the E. coli lac Z gene (lacZ'), encoding the N-terminal 375 amino acids of Beta-galactosidase (Beta-gal). cDNA clones encoding chain 1 and chain 2 of TRFP were altered using PCR methods such that the 5' end possessed an in-frame poly-histidine sequence followed by an asp-pro acid-sensitive bond. Cultures containing the chain 1 or 2 expression constructs produce substantial quantities of recombinant fusion protein products upon IPTG induction. The presence of the poly-histidine reporter group has allowed the recombinants to be highly purified using immobilized metal-ion affinity chromatography (Hochuli, E. et al. BioTechnology 6: 1321-1325 (1988). Mild-acid cleavage of the Asp-Pro site leads to the release of intact full-length TRFP chain 1 or chain 2 protein. Standard protein purification methods lead to substantial quantities of recombinant protein free of irrelevant sequences. Protein sequence analysis of the purified peptide have verified the authenticity of the sequence. Rabbit anti-peptide antisera directed against either chain 1 sequence (Fel 1, EITPAVKRDVDLFLTGT; Fel 2, DVDLFLTGTPDEYVEQV; Fel 4, NARILKNCVDAKMTEEDKE), or chain 2 sequences (Fel 18, LLLDLSLTKVNATEPERTAMKKIQDC), have been generated. The anti-peptide antisera react with the recombinant proteins (described above) on Western blots.

Recombinant chain 1 and chain 2 peptides, and fragments or modifications thereof, can be used as desensitizing therapeutants.

### EXAMPLE 5. T cell Studies with Purified T Cell Reactive Protein

Peripheral blood mononuclear cells (PBMC) were purified from 60 ml of heparinized blood from cat allergic patients. PBMC were subsequently treated as described below, although in individual cases, the length of time of cultivation with IL-2 and/or IL-4 and the specific peptides used for stimulation varied.

10 ml of PBMC from patient 131 at 10⁶/ml were cultured at 37°C for 7 days in the presence of 5 micrograms purified TRFP/ml RPMI-1640 supplemented with 5% pooled human AB serum. Viable cells were purified by Ficoll-Hypaque centrifugation and cultured for three weeks at 5 units recombinant human IL-2/ml and 5 units recombinant human IL-4/ml. The resting T cells were then restimulated (secondary) with 5 micrograms TRFP at 2 x 10⁵/ml in the presence of irradiated (3500 Rads) autologous PBMC at a concentration of 5 x 10⁵/ml for three days, purified by Ficoll-Hypaque centrifugation and grown in 5 units IL-2 and 5 units IL-4/ml for two weeks. For assay, 2 x 10⁴ resting secondary T cells were restimulated (tertiary) in the presence of 5 x 10⁴ irradiated (3500 Rads) autologous PBMC or 2 x 10⁴ transformed B cells (20,000 Rads) with various concentrations of allergens or their fragments in a volume of 200 microliters in a 96-well round bottom assay plates for 3 days. Each well then received 1 microCurie tritiated (methyl) thymidine for 16 hours. The counts incorporated were collected onto glass fiber filters and processed for liquid scintillation counting.

Antigens used: T cell reactive feline protein (TRFP), Hollister-Stier cat epithelium skin test reagent (CST), IPC ragweed pollen extract (pollen), and synthetic peptides derived from the TRFP protein sequence (See Figures 6 and 7). Significant T cell proliferation is generally regarded as greater than 2.5 times the media control (T cells and antigen presenting cells alone).

Alternatively, PBMC were treated as follows: the primary stimulated cells were cultured in IL-2/IL-4 for two weeks. The resting T cells derived from this culture were tested in a secondary assay with some, but not all, of the above allergens. The results of these assessments are shown in Tables 2 and 3. Figure 9 demonstrates that T cells from patient 131 respond to T cell epitopes present in the Fel 1, 5 and 8 peptides. This type of epitope analysis has allowed the definition of T cell epitopes present in TRFP. Using a larger panel of patients, we have demonstrated the dominant epitopes in a heterogeneous population by deriving a positivity index (PI). The PI is derived from the average stimulation index of the responding population multiplied by the percentage of individuals that demonstrate a positive response to that peptide. This analysis is shown in Table 2 and 3.

This data demonstrates that while most of the TRFP protein contains T cell epitopes capable of stimulating T cells from some individuals, there are major differences in the strength of the elicited T cell response obtained with different portions of the TRFP molecule. The data has shown that each epitope works in some individuals and that each individual has a characteristic response pattern. For example, it appears that Fel 28 is the weakest T cell epitope containing region of TRFP (Table 3), whereas the most dominant T-cell epitopes are contained by the Fel 8 peptide (Table 2).

**Table 2**

| Response of Cat Allergic Patients to Chain #1 TRFP Peptides | | | | |
|---|---|---|---|---|
| Peptide | Amino acid | PI¹ | SI² | N³ |
| Fel 1⁴ | 1 - 17, 3T | 392 | 5.6 | 121 |
| 1-2 | 1 - 17 | 311 | 7.4 | 83 |
| 1-3 | 4 - 17 | 422 | 6.2 | 26 |
| 1-4 | 6 - 17 | 816 | 9.6 | 32 |
| 1-5 | 8 - 17 | 286 | 5.2 | 25 |
| 1-6 | 10 - 17 | 312 | 5.2 | 26 |
| | | | | |
| Fel 2 | 9 - 25 | 416 | 7.3 | 30 |
| | | | | |
| Fel 3⁶ | 18 - 33, | 674 | 9.5 | 123 |
| | 31P,32D | | | |
| 3-1 | 18 - 33 | 638 | 9.4 | 40 |
| 3-10 | 18 - 31 | 504 | 6.9 | 28 |
| 3-11 | 18 - 30 | 863 | 10.4 | 12 |
| 3-15 | 18 - 29 | 1040 | 10.4 | 13 |
| 3-13 | 18 - 28 | 690 | 11.5 | 14 |
| 3-14 | 18 - 27 | 260 | 6.2 | 10 |
| | | | | |
| Fel 8 | 1 - 30 | 1393 | 18.1 | 86 |
| 8-1 | 5 - 33 | 1374 | 15.1 | 47 |
| 8-2 | 6 - 33 | 1353 | 15.2 | 47 |
| 8-3 | 7 - 33 | 1437 | 16.9 | 47 |
| | | | | |
| Fel 14 | 18 - 43 | 1054 | 13.7 | 125 |
| 14-1 | 23 - 36 | 871 | 9.9 | 88 |
| | | | | |
| 14-3 | 25 - 36 | 621 | 6.9 | 90 |
| 14-4 | 26 - 36 | 474 | 6.0 | 79 |
| 14-5 | 27 - 36 | 286 | 5.4 | 53 |
| 14-2 | 29 - 42 | 336 | 5.6 | 60 |
| | | | | |
| Fel 4 | 37 - 55 | 601 | 7.7 | 120 |
| 4-1 | 37 - 52 | 822 | 9.9 | 20 |
| 4-2 | 37 - 49 | 378 | 6.2 | 15 |
| 4-3 | 37 - 46 | 185 | 3.7 | 13 |
| | | | | |
| Fel 30-1 | 25 - 49 | 268 | 5.6 | 23 |
| 30-2 | 25 - 48 | 248 | 4.2 | 22 |
| 30-3 | 25 - 47 | 230 | 4.8 | 23 |
| 30-4 | 29 - 55 | 1079 | 11.6 | 44 |
| 30-5 | 29 - 54 | 792 | 11.0 | 43 |
| 30-6 | 29 - 53 | 415 | 6.2 | 43 |
| 30-7 | 26 - 55 | 339 | 5.3 | 14 |
| 30-8 | 28 - 55 | 262 | 4.1 | 14 |
| | | | | |
| Fel 15 | 44 - 60 | 440 | 11.0 | 60 |
| | | | | |
| Fel 23 | 51 - 66 | 343 | 6.6 | 63 |
| | | | | |
| Fel 21⁵ | 56 - 70, 70R | 360 | 5.8 | 66 |

| | | | | |
|---|---|---|---|---|
| ¹PI: Average SI of all responding patients tested multiplied by the percent of those patients with a positive response | | | | |
| ²SI: Average of the cpm of T cell and antigen presenting cell proliferation to the antigen divided by cpm of T cells and antigen presenting cells alone from responding patients. An SI of ≥ 2.5 is considered positive. | | | | |
| ³N: Number of patients tested. | | | | |
| ⁴: Amino acid 3 changed to T. | | | | |
| ⁵: Amino acid 70 changed to R. | | | | |
| 6: Amino acids 31 and 32 changed to P and D, respectively. | | | | |

**Table 3**

| Response of Cat Allergic Patients to Chain #2 TRFP peptide | | | | |
|---|---|---|---|---|
| Peptide | Amino acid | PI | SI | N |
| Fel 16 | 1 - 22 | 283 | 5.9 | 52 |
| | | | | |
| Fel 17 | 12 - 33 | 421 | 6.1 | 114 |
| | | | | |
| Fel 32-1 | 12 - 24 | 442 | 6.6 | 21 |
| 32-2 | 14 - 24 | 424 | 5.3 | 20 |
| 32-3 | 16 - 24 | 270 | 3.7 | 22 |
| | | | | |
| Fel 18 | 23 - 48 | 466 | 6.3 | 99 |
| | | | | |
| Fel 33-1 | 26 - 36 | 340 | 5.4 | 63 |
| 33-2 | 26 - 38 | 210 | 4.2 | 50 |
| 33-3 | 26 - 40 | 235 | 5.0 | 47 |
| | | | | |
| Fel 31-1 | 14 - 40 | 733 | 9.4 | 36 |
| 31-2 | 14 - 39 | 599 | 8.1 | 35 |
| 31-3 | 14 - 38 | 598 | 8.3 | 36 |
| 31-4 | 14 - 37 | 622 | 8.4 | 35 |
| 31-5 | 14 - 36 | 539 | 7.6 | 37 |
| 31-6 | 15 - 40 | 295 | 4.4 | 33 |
| 31-7 | 15 - 36 | 267 | 5.8 | 33 |
| | | | | |
| Fel 20-1 | 34 - 59 | 395 | 5.9 | 79 |
| | | | | |
| Fel 25 | 49 - 68 | 350 | 7.6 | 56 |
| | | | | |
| Fel 28 | 60 - 82 | 94 | 3.6 | 43 |
| | | | | |
| Fel 28-1¹ | 60 - 82 | 176 | 5.5 | 44 |
| | | | | |
| Fel 29 | 74 - 92 | 259 | 5.5 | 47 |

| | | | | |
|---|---|---|---|---|
| ¹ Based on short form chain 2 sequence (C2S) | | | | |

### EXAMPLE 6. Induction of T cell anergy by a TRFP T cell epitope containing peptide

Exposure of peptide specific T cells to their specific peptide can induce anergy to the protein containing the T cell epitopes (Jenkins, M.K., and Schwartz, R.H. J. Exp. Med 165:302-319 (1987)). It is predicted that any strong T cell epitope can be used to induce tolerance to the whole allergen. This would result in the inability of the individual to respond to a natural allergen exposure. The individual would not respond by the stimulation of helper T cells. The lack of helper T cells would result in an altered lymphokine response and/or the absence of an IgE response and, consequently, a reduced allergic response to cat allergens.

Patient 155 TRFP secondary primed T cells (2.5 x 10⁶) were rested and cultured with (2.5 x 10⁶) irradiated autologous Epstein Barr Virus transformed B cells (EBV) in 1 ml of complete RPMI with 10% AB serum in 12x75mm polypropylene snap cap tubes and increasing amounts of antigen over 5 consecutive days. The T cell cultures were exposed to 5µg/ml TRFP on day 0 and 5µg/ml, 10µg/ml, 10µg/ml and 20µg/ml thereafter. The peptide treated cultures were exposed to 1µg/ml peptide on day 0 and 1µg/ml, 1µg/ml, 2µg/ml and 5µg/ml thereafter. In addition, 0.5ml of fresh media was replaced on day 2. The cells were then washed and set up for a proliferation assay with 2x10⁴ T cells and 2x10⁴ irradiated (2500 Rad) autologous EBV and various doses of antigen. T cell proliferation was measured as incorporation of tritiated thymidine at day 9. The induction of in vitro anergy or tolerance is demonstrated in Table 4. This experiment demonstrates the ability of TRFP and peptides thereof to induce anergy or tolerance in antigen specific T cell lines.

**Table 4**

| Antigen Response of a TRFP-primed T cell culture exposed to a Fel 8-3 or TRFP. | | | | |
|---|---|---|---|---|
| | | T cell proliferation (cpm) following Antigenic challenge: | | |
| Tolerance treatment: | - | TRFP | Fel 8-3 | Ragweed Peptide |
| - | 1,530 | 58,890 | 58,150 | 2,130 |
| Fel 8-3 | 1,270 | 12,320 | 3,850 | 5,130 |
| TRFP | 2,190 | 33,160 | 36,030 | 6,670 |
| Ragweed Pep. | 920 | 64,050 | 45,020 | 3,590 |

### EXAMPLE 7. Cytokine profiles of T cells responsive to purified TRFP or synthetic peptides derived from the TRFP protein sequence

Peripheral blood mononuclear cells (PBMC) were purified from cat-allergic patients as described in Example 5. Five x 10⁶ PBMC were cultured at 2 x 10⁶/ml for 36 hours in the presence of medium only, 20 micrograms purified TRFP/ml or 50 micrograms peptide/ml. The cells were then washed two times with phosphate buffered saline (PBS, pH 7.2) and lysed with 2 ml 0.4 M guanidinium isothiocyanate, 0.5% Sarkosyl, 5 mM sodium citrate, 0.1 M 2-mercaptoethanol, pH 7.0. The lysate was then forced through a 26 gauge needle to shear genomic DNA, and was layered onto a 2 ml cushion of 5.7 M CsCl, 0.01 M EDTA, pH 7.5 in diethylpyrocarbonate (DEPC)-treated water. The lysate was centrifuged at 35,000 RPM in a Beckman SW41 rotor for 18 hours at 20°C. The RNA pellet was resuspended in 0.4 ml TE (10 mM Tris, 1 mM EDTA) pH 7.5, 0.1% SDS, and then extracted three times with 0.5 ml phenol/0.2 ml chloroform. The RNA was then precipitated on dry ice with 2.5 volumes ice-cold ethanol, 1/10 volume 3 M sodium acetate, pH 5.2 in DEPC-treated water, rinsed once with 70% ethanol in DEPC-treated water, and dried. The RNA pellet was resuspended in 2 microliters TE, pH 7.5 in DEPC-treated water.

Total cellular RNA was converted to cDNA using the Superscript kit (BRL, Bethesda, MD). Two microliters of RNA were added to one microliter oligo (dT)₁₂₋₁₈ (500 micrograms/ml) and 9 microliters water. The sample was heated to 70°C for 10 minutes and ice quenched. Four microliters 5X buffer were added to the sample along with 2 microliters 0.1 M DTT and 1 microliter dNTP mix (10 mM each, dATP, dCTP, dGTP, dTTP). One microliter reverse transcriptase (200U) was added and the reaction was carried out for one hour at 42°C. The reaction was terminated by incubation of the sample at 90°C for five minutes and stored at -80°C.

Ten-fold serial dilutions of T cell cDNA in 10 mM TRIS, pH 7.5 were amplified using the standard kit and protocol recommended by Perkin Elmer-Cetus (Redwood City, CA). Each sample received 26.65 microliters water, 5 microliters 10X PCR buffer, 8 microliters of dNTP mix (1.25 mM each of dATP, dCTP, dGTP and dTTP), 0.1 microliter alpha ³²P-dCTP (3000 Ci/mmol), 0.25 microliter AmpliTaq, 5 microliters cDNA and 5 microliters cytokine-specific 5' and 3' primers (20 micromolar). The primers used for most cytokines and the beta-actin control were purchased from Clontech (Redwood City, CA). The human IL-4 primers were purchased from Research Genetics (Huntsville, AL) and had the following sequences:

5' hIL-4 primer
5'-GTC-CAC-GGA-CAC-AAG-TGC-GAT-ATC-ACC-3'
3' hIL-4 primer
5'-GTT-GGC-TTC-CTT-CAC-AGG-ACA-GGA-ATT-C-3'

The reactions were carried out after overlaying each sample with one drop of mineral oil, with the following program in a programmable thermal cycler (MJ Research, Cambridge, MA).

| Step | Temperature | Time |
|---|---|---|
| 1 | 94°C | 1 min |
| 2 | 60°C | 1 min |
| 3 | 72°C | 2 min |
| 4 | cycle to step 1 | 29 times |
| 5 | 72°C | 7 min |
| 6 | 4°C | hold |

The PCR products were extracted once with 25 microliters chloroform and 25 microliters of each sample were then electrophoresed on an 8% polyacrylamide gel at 250 V. The gel was dried and exposed to pre-flashed x-ray film. Several exposures of each gel were then scanned using a Shimadzu flying spot laser densitometer. Values on the linear portion of the titrations were then compared to the medium control values to obtain a stimulation index for each sample. Primers for beta-actin were included as a control for general cDNA levels in each sample. Where the medium control values are not detectable, the lowest measurable response was set at 1.00. In other experiments, levels of cytokine cDNA can be compared directly with previously amplified cytokine-specific cDNAs as standards. Thus, absolute levels of particular cytokine cDNAs can be compared from one sample to another and from one experiment to another. Results of a representative experiment are shown in Table 5. In this experiment, IL-2, IL-4 and IFN-gamma levels were measured. As shown, in this particular cat-allergic patient, peptides such as Fel 18 generate more IL-4 than certain other TRFP-derived peptides (Fel 14 and Fel 17). This analysis will be expanded to studies of other cytokines involved in the generation of allergic responses, such as IL-5, IL-8, IL-9, TGF-beta. Samples of cDNA from each treatment can also be saved for later analysis once additional cytokines are identified and sequenced. Peptides generating a spectrum of cytokines favorable for the generation of allergic responses can be avoided for therapeutic use in the treatment of cat allergy. Similarly, TRFP-derived peptides that are shown to generate cytokines which dampen the allergic response, such as IFN-gamma and IL-10, can be selected for treatment of cat allergy.

**Table 5**

| IL-2, IFN-γ and IL-4 Measurements by PCR | | | | | |
|---|---|---|---|---|---|
| Patient 409 primary 36 hour culture | | | | | |
| | **STIMULATION INDEX** | | | **RATIO** | |
| Treatment | IL-2 | IFN-γ | IL-4 | IL-2/IL-4 | IFN/IL-4 |
| Medium | -- | 1.0 | 1.0 | | |
| TRFP | -- | 2.1 | 3.8 | | 0.9 |
| Fel 8 | 1.0 | 1.8 | 0.3 | 3.3 | 6.0 |
| Fel 14 | 106.9 | 75.0 | 1.9 | 56.3 | 39.5 |
| Fel 4 | -- | 25.8 | 10.0 | | 2.6 |
| Fel 21 | 23.9 | 41.6 | 3.2 | 7.5 | 13.0 |
| | | | | | |
| Fel 17 | 315.1 | 82.4 | 7.5 | 42.0 | 11.0 |
| Fel 18 | 4.6 | 5.6 | 12.0 | 0.4 | 0.5 |
| Fel 20-1 | -- | 12.0 | 4.6 | | 2.6 |
| Fel 25 | 2.9 | 12.7 | 2.5 | 1.2 | 5.1 |
| Fel 28 | -- | 3.8 | 0.5 | | 7.6 |
| Fel 29 | 1.2 | 3.0 | 1.1 | 1.1 | 2.7 |

## Claims

1. A composition for use in medicine, comprising a first polypeptide that:
a) has an amino acid sequence as shown in any of Figures 3, 4, 5 or 7, or
b) has at least part of a sequence as described in a) above and has an epitope in common therewith, or
c) is a modified form of a polypeptide as described in a) or b) above, but has at least one epitope in common therewith;
wherein said composition can be used to reduce an allergic response to a cat antigen in an individual sensitive to said antigen.

2. A composition for use in medicine according to claim 1; wherein the first polypeptide is a polypeptide as described in a) or b) of claim 1.

3. A composition for use in medicine according to claim 1 or claim 2, wherein the first polypeptide is a peptide as shown in Table 3.

4. A composition for use in medicine according to any of claims 1 to 3, comprising a mixture of said first polypeptides.

5. A composition for use in medicine according to any preceding claim, further comprising a second polypeptide that is different from the first polypeptide and that:
a) has an amino acid sequence as shown in Figure 1, Figure 2 or Figure 6, or
b) has at least part of a sequence as described in a) above and has an epitope in common therewith, or
c) is a modified form of a polypeptide as described in a) or b) above, but has at least one epitope in common therewith.

6. A composition for use in medicine according to claim 5, wherein the second polypeptide is a polypeptide as described in a) or b) of claim 4.

7. A composition for use in medicine according to claim 5 of claim 6, wherein the second polypeptide is a peptide as shown in Table 2.

8. A composition for use in medicine according to any of claims 5 to 7, comprising a mixture of said second polypeptides, or a mixture of said first and second polypeptides.

9. A composition for use in medicine according to any of claims 5 to 8, wherein the first and second polypeptide are covalently linked in the form of a heterodimer.

10. A composition for use in medicine according to any preceding claim, wherein said composition has a stimulation index of at least approximately 4.0 or a positivity index of at least approximately 250, wherein the stimulation index is defined as the ratio of measured proliferation of T cells and antigen presenting cells from a responding individual when exposed to antigen to the measured proliferation of said cells in the absence of antigen, and wherein the positivity index is defined as the average stimulation index of a population of responding individuals tested multiplied by the percentage of individuals in the population exhibiting a positive response.

11. A polypeptide that:
a) has an amino acid sequence as shown in any of Figures 3, 4, 5 or 7, or
b) has at least part of a sequence as described in a) above and has an epitope in common therewith, or
c) is a modified form of a polypeptide as described in a) or b) above, but has at least one epitope in common therewith.

12. A polypeptide according to claim 11 that has an amino acid sequence as shown in any of Figures 3, 4, 5 or 7, or has at least part of said sequence.

13. A polypeptide according to claim 11 or 12 that is a peptide as shown in Table 3.

14. A polypeptide according to any of claims 11 to 13, wherein the polypeptide has a stimulation index of at least approximately 4.0 or a positivity index of at least approximately 250 wherein the stimulation index is defined as the ratio of measured proliferation of T cells and antigen presenting cells from a responding individual when exposed to antigen to the measured proliferation of said cells in the absence of antigen, and wherein the positivity index is defined as the average stimulation index of a population of responding individuals tested multiplied by the percentage of individuals in the population exhibiting a positive response.

15. A polypeptide according to any of claims 11 to 13, for use in medicine.

16. The use of a polypeptide as described in any of claims 1 to 14, or of a composition as described in any of claims 1 to 10, in the preparation of a medicament for treating a cat allergy.

17. A polypeptide as described in any of claims 1 to 14, or a composition as described in any of claims 1 to 10, for use in diagnosis.

18. An *ex vivo* method of detecting sensitivity in an individual to a cat allergen, comprising combining a blood sample obtained from the individual with a polypeptide as described in any of claims 1 to 14 or with a composition as described in any of claims 1 to 10.

19. A method comprising preparing a polypeptide as described in any of claims 11 to 13 by recombinant means.

20. A method according to claim 19, further comprising preparing a second polypeptide as described in any of claims 5 to 7 by recombinant means.

21. A method according to claim 19 or 20, comprising expressing said polypeptide or polypeptides in *E. coli*.

22. A method according to claim 20 or 21, which is used to produce a heterodimer as described in claim 9.

23. DNA encoding a polypeptide as described in any of claims 11 to 14.

24. DNA according to claim 23 in the form of cDNA.

25. DNA capable of hybridizing to DNA according to claim 23 or 24.

## Patentansprüche

1. Zusammensetzung zur Verwendung in der Medizin, die ein erstes Polypeptid umfaßt, das
a) eine wie in einer der Figuren 3, 4, 5 oder 7 gezeigte Aminosäuresequenz aufweist, oder
b) zumindest einen Teil einer Sequenz wie in a) oben beschrieben aufweist und ein Epitop damit gemeinsam hat, oder
c) eine modifizierte Form eines. wie in a) oder b) oben beschriebenen Polypeptids ist, jedoch zumindest ein Epitop damit gemeinsam hat;
wobei die Zusammensetzung zur Reduktion einer allergischen Reaktion auf ein Katzen-Antigen bei einem Individuum verwendet werden kann, das gegenüber dem Antigen empfindlich ist.

2. Zusammensetzung zur Verwendung in der Medizin nach Anspruch 1, wobei das erste Polypeptid ein wie in a) oder b) von Anspruch 1 beschriebenes Polypeptid ist.

3. Zusammensetzung zur Verwendung in der Medizin nach Anspruch 1 oder Anspruch 2, wobei das erste Polypeptid ein wie in Tabelle 3 dargestelltes Polypeptid ist.

4. Zusammensetzung zur Verwendung in der Medizin nach einem der Ansprüche 1 bis 3, die ein Gemisch der ersten Polypeptide umfasst.

5. Zusammensetzung zur Verwendung in der Medizin nach irgend einem vorhergehenden Anspruch, die weiterhin ein zweites Polypeptid umfaßt, das von dem ersten Polypeptid verschieden ist und das:
a) eine Aminosäuresequenz wie in der Figur 1, Figur 2 oder Figur 6 dargestellt aufweist, oder
b) zumindest einen Teil einer Sequenz wie in a) oben beschrieben aufweist und ein Epitop mit dieser gemeinsam hat; oder
c) eine modifizierte Form eines wie in a) oder b) oben beschriebenen Polypeptids ist, jedoch zumindest ein Epitop damit gemeinsam hat.

6. Zusammensetzung zur Verwendung in der Medizin nach Anspruch 5, wobei das zweite Polypeptid ein wie in a) oder b) von Anspruch 4 beschriebenes Polypeptid ist.

7. Zusammensetzung zur Verwendung in der Medizin nach Anspruch 5 oder Anspruch 6, wobei das zweite Polypeptid ein wie in Tabelle 2 dargestelltes Peptid ist.

8. Zusammensetzung zur Verwendung in der Medizin nach einem der Ansprüche 5 bis 7, die ein Gemisch der zweiten Polypeptide oder ein Gemisch der ersten und zweiten Polypeptide umfasst.

9. Zusammensetzung zur Verwendung in der Medizin nach einem der Ansprüche 5 bis 8, wobei das erste und zweite Polypeptid in Form eines Heterodimers kovalent gebunden ist.

10. Zusammensetzung zur Verwendung in der Medizin nach irgend einem vorhergehenden Anspruch, wobei die Zusammensetzung einen Stimulationsindex von zumindest ungefähr 4,0 oder einen Positivitätsindex von zumindest ungefähr 250 aufweist, wobei der Stimulationsindex als das Verhältnis der gemessenen Proliferation von T-Zellen und Antigen-präsentierenden Zellen von einem reagierenden Individuum, wenn es gegenüber dem Antigen ausgesetzt ist, gegenüber der gemessenen Proliferation der Zellen bei Fehlen des Antigens definiert ist, und wobei der Positivitätsindex als der durchschnittliche Stimulationsindex einer Population von reagierenden Individuen, die getestet wurden, multipliziert mit dem Prozentsatz der Individuen in der Population, die eine positive Reaktion zeigen, definiert ist.

11. Polypeptid, das:
a) eine wie in einer der Figuren 3, 4, 5 oder 7 gezeigte Aminosäuresequenz aufweist oder
b) zumindest einen Teil einer Sequenz wie oben in a) beschrieben aufweist und damit ein Epitop gemeinsam hat, oder
c) eine modifizierte Form eines wie in a) oder b) oben beschriebenen Polypeptids ist, jedoch zumindest ein Epitop damit gemeinsam hat.

12. Polypeptid nach Anspruch 11, das eine wie in irgend einer der Figuren 3, 4, 5 oder 7 dargestellte Aminosäuresequenz aufweist, oder zumindest einen Teil der Sequenz aufweist.

13. Polypeptid nach Anspruch 11 oder 12, das ein wie in Tabelle 3 dargestelltes Peptid ist.

14. Polypeptid nach einem der Anspruche 11. bis 13, wobei das Polypeptid einen Stimulationsindex von zumindest ungefähr 4,0 oder einen Positivitätsindex von zumindest ungefähr 250 aufweist, wobei der Simulationsindex als das Verhältnis der gemessenen Proliferation von T-Zellen und Antigen präsentierenden Zellen von einem reagierenden Individuum, wenn es einem Antigen ausgesetzt wird, zur gemessenen Proliferation der Zellen bei Fehlen eines Antigens definiert ist und wobei der Positivitätsindex als der durchschnittliche Stimulationsindex einer Population reagierender Individuen, die getestet wurden, multipliziert mit dem Prozentsatz der Individuen in der Population, die eine positive. Reaktion zeigen, definiert ist.

15. Polypeptid nach einem der Ansprüche 11 bis 13 zur Verwendung in der Medizin.

16. Verwendung eines Polypeptids wie in einem der Ansprüche 1 bis 14 beschrieben oder einer Zusammensetzung wie in einem der Ansprüche 1 bis 10 beschrieben bei der Herstellung eines Medikamentes zur Behandlung einer Katzenallergie.

17. Polypeptid wie in einem der Ansprüche 1 bis 14 beschrieben oder eine Zusammensetzung wie in einem der Ansprüche 1 bis 10 beschrieben, zur Verwendung in der Diagnose.

18. Ex vivo Verfahren zum Nachweisen der Sensitivität in einem Individuum gegenüber einem Katzen-Allergen, umfassend das Vereinigen einer Blutprobe, die von dem Individuum gewonnen wurde, mit einem wie in einem der Ansprüche 1 bis 14 beschriebenen Polypeptid oder mit einer Zusammensetzung, wie in einem der Ansprüche 1 bis 10 beschrieben.

19. Verfahren, das das Herstellen eines Polypeptids wie in einem der Ansprüche 11 bis 13 beschrieben durch rekombinante Mittel umfaßt.

20. Verfahren nach Anspruch 19, das weiter die Herstellung eines zweiten wie in einem der Ansprüche 5 bis 7 beschriebenen Polypeptids durch rekombinante Mittel umfaßt.

21. Verfahren nach Anspruch 19 oder 20, das das Exprimieren des Polypeptids oder der Polypeptide in E. coli umfasst.

22. Verfahren nach Anspruch 20 oder 21, das zur Erzeugung eines Heterodimers, wie in Anspruch 9 beschrieben, verwendet wird.

23. DNA, die ein Polypeptid wie in einem der Ansprüche 11 bis 14 beschrieben kodiert.

24. DNA nach Anspruch 23 in Form von cDNA.

25. DNA, die zum Hybridisieren an DNA nach Anspruch 23 oder 24 in der Lage ist.

## Revendications

1. Une composition pour une utilisation en médecine, comprenant un premier polypeptide qui:
a) possède une séquence d'acides aminés telle que montrée dans l'une quelconque des figures 3, 4, 5 ou 7, ou
b) possède au moins une partie d'une séquence telle que décrite dans a) ci-dessus et possède un épitope en commun avec celle-ci, ou
c) est une forme modifiée d'un polypeptide tel que décrit dans a) ou b) ci-dessus, mais possède au moins un épitope en commun avec celui-ci;
où cette composition peut être utilisée pour réduire une réponse allergique à un antigène de chat chez un individu sensible à cet antigène.

2. Composition pour une utilisation en médecine selon la revendication 1; dans laquelle le premier polypeptide est un polypeptide tel que décrit dans a) ou b) de la revendication 1.

3. Composition pour une utilisation en médecine selon la revendication 1 ou 2, dans laquelle le premier polypeptide est un peptide comme montré dans le tableau 3.

4. Composition pour une utilisation en médecine, selon l'une quelconque des revendications 1 à 3, comprenant un mélange de ces premiers polypeptides.

5. Composition pour une utilisation en médecine selon l'une quelconque des revendications précédentes, comprenant en outre un second polypeptide qui est différent du premier polypeptide et qui:
a) possède une séquence d'acides aminés telle que montrée dans la figure 1, figure 2 ou figure 6, ou
b) possède au moins une partie d'une séquence telle que décrite dans a) ci-dessus et possède un épitope en commun avec celle-ci, ou
c) est une forme modifiée d'un polypeptide tel que décrit dans a) ou b) ci-dessus, mais possède au moins un épitope en commun avec celui-ci.

6. Composition pour une utilisation en médecine selon la revendication 5, dans laquelle le second polypeptide est un polypeptide tel que décrit dans a) ou b) de la revendication 4.

7. Composition pour une utilisation en médecine selon la revendication 5 ou 6, dans laquelle le second polypeptide est un peptide tel que montré dans le tableau 2.

8. Composition pour une utilisation en médecine selon l'une quelconque des revendications 5 à 7, comprenant un mélange de ces seconds polypeptides, ou un mélange de ces premier et second polypeptides.

9. Composition pour une utilisation en médecine selon l'une quelconque des revendications 5 à 8, dans laquelle les premiers et seconds polypeptides sont liés de manière covalente sous forme d'un hétérodimère.

10. Composition pour une utilisation en médecine selon l'une quelconque des revendications précédentes, dans laquelle cette composition possède un indice de stimulation d'au moins approximativement 4,0 ou un indice de positivité d'au moins approximativement 250, dans laquelle l'indice de stimulation est défini sous forme du rapport de la prolifération mesurée des cellules T et des cellules présentant un antigène provenant de la réponse d'un individu lorsqu'il est exposé à un antigène en fonction de la prolifération mesurée de ces cellules en l'absence d'antigène, et dans laquelle l'indice de positivité est défini comme l'indice de stimulation moyen d'une population d'individus testés donnant une réponse multiplié par le pourcentage d'individus dans la population manifestant une réponse positive.

11. Un polypeptide qui:
a) possède une séquence d'acides aminés telle que montrée dans l'une quelconque des figures 3, 4, 5 ou 7, ou
b) possède au moins une partie d'une séquence telle que décrite dans a) ci-dessus et possède un épitope en commun avec celle-ci, ou
c) est une forme modifiée d'un polypeptide tel que décrit dans a) ou b) ci-dessus, mais possède au moins un épitope en commun avec celui-ci.

12. Un polypeptide selon la revendication 11 qui possède une séquence d'acides aminés comme montrée dans l'une quelconque des figures 3, 4, 5 ou 7, ou qui possède au moins une partie de cette séquence.

13. Un polypeptide selon la revendication 11 ou 12 qui est un peptide tel que montré dans le tableau 3.

14. Un polypeptide selon l'une quelconque des revendications 11 à 13, dans lequel le polypeptide possède un indice de stimulation d'au moins approximativement 4,0 ou un indice de positivité d'au moins approximativement 250 dans lequel l'indice de stimulation est défini comme le rapport d'une prolifération mesurée de cellules T et de cellules présentant un antigène provenant d'un individu donnant une réponse lorsqu'il est exposé à un antigène en fonction d'une prolifération mesurée de ces cellules en l'absence d'un antigène, et dans lequel l'indice de positivité est défini comme l'indice de stimulation moyen d'une population d'individus testés donnant une réponse multiplié par le pourcentage d'individus dans la population manifestant une réponse positive.

15. Un polypeptide selon l'une quelconque des revendications 11 à 13, pour une utilisation en médecine.

16. Utilisation d'un polypeptide tel que décrit dans l'une quelconque des revendications 1 à 14, ou d'une composition telle que décrite dans l'une quelconque des revendications 1 à 10, dans la préparation d'un médicament pour le traitement d'une allergie aux chats.

17. Un polypeptide tel que décrit dans l'une quelconque des revendications 1 à 14, ou une composition telle que décrite dans l'une quelconque des revendications 1 à 10, pour une utilisation dans un diagnostic.

18. Une méthode *ex vivo* pour détecter chez un individu une sensibilité à un allergène de chat, comprenant la combinaison d'un échantillon de sang obtenu d'un individu avec un polypeptide tel que décrit dans l'une quelconque des revendications 1 à 14 ou avec une composition telle que décrite dans l'une quelconque des revendications 1 à 10.

19. Une méthode comprenant la préparation d'un polypeptide tel que décrit dans l'une quelconque des revendications 11 à 13 par un moyen recombinant.

20. Une méthode selon la revendication 19, comprenant en outre la préparation d'un second polypeptide tel que décrit dans l'une quelconque des revendications 5 à 7 par un moyen recombinant.

21. Une méthode selon la revendication 19 ou 20, comprenant l'expression de ce polypeptide ou polypeptides chez *E. coli*.

22. Une méthode selon la revendication 20 ou 21, qui est utilisée pour produire un hétérodimère tel que

23. ADN codant pour un polypeptide tel que décrit dans l'une quelconque des revendications 11 à 14.

24. ADN selon la revendication 23 sous la forme d'un ADNc.

25. ADN capable de s'hybrider à l'ADN selon la revendication 23 ou 24.
